# FASCICULE DE BREVET EUROPEEN

(11) **EP 0 728 466 B1**
(45) Date de publication et mention de la délivrance du brevet: **14.06.2000**
(21) Numéro de dépôt: 96400287.7
(22) Date de dépôt: 12.02.1996
(51) Int. Cl.: A61K 7/13

(54) **Composition pour la teinture d'oxydation des fibres kératiniques comprenant une base d'oxydation, un coupleur indolique et un coupleur hétérocyclique additionnel, et procédé de teinture**
Zusammensetzung zur oxidativen Färbung von keratinischen Fasern, die eine Oxidationsbase, einen Indol-Kuppler und einen zusätzlichen heterozyklischen Kuppler enthält, sowie Färbeverfahren
Keratinous fibers oxidation dyeing composition comprising an oxidation base, an indolic coupler and an additional heterocyclic coupler and dyeing process

(30) Priorité: 27.02.1995 FR 9502270
(43) Date de publication de la demande: 28.08.1996
(73) Titulaire: L'OREAL, 75008 Paris (FR)
(72) Inventeur: Audousset, Marie-Pascale, F-92600 Asnieres (FR); Cotteret, Jean, F-78480 Verneuil sur Seine (FR)
(74) Mandataire: Goulard, Sophie

(56) Documents cités:
- EP-A- 0 465 339
- EP-A- 0 465 340
- DE-A- 3 743 769
- DE-A- 3 930 446

## Description

La présente invention a pour objet une composition pour la teinture d'oxydation des fibres kératiniques, en particulier des fibres kératiniques humaines telles que les cheveux, comprenant une base d'oxydation en association avec d'une part au moins un coupleur indolique convenablement sélectionné et, d'autre part, au moins un coupleur hétérocyclique additionnel également convenablement sélectionné, ainsi que le procédé de teinture mettant en oeuvre cette composition avec un agent oxydant.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques tels que des dérivés de diaminopyrazole, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des dérivés indoliques et en particulier le 4-hydroxyindole.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, notamment dans les demandes de brevet allemand DE 3 031 709, DE 3 743 769, français FR 2 362 112 et européen EP 459 901, des compositions pour la teinture d'oxydation des fibres kératiniques contenant une base d'oxydation en association avec un coupleur hétérocyclique tel que par exemple le 4-hydroxy 1,2-méthylènedioxy benzène, le 4-amino 1,2-méthylènedioxy benzène, le 4-hydroxy indole ou la 6-hydroxybenzomorpholine. De telles compositions permettent d'atteindre des gammes variées de nuances mais elles ne sont cependant pas entièrement satisfaisantes notamment du point de vue de la tenue des colorations obtenues vis à vis des diverses agressions que peuvent subir les cheveux.

Il a également déjà été proposé, notamment dans les demandes de brevet EP 0 465 339 ou EP 0 465 340, des procédés de teinture d'oxydation des fibres kératiniques à pH acide mettant en oeuvre des compositions renfermant une base d'oxydation, du 4-hydroxyindole ou l'un de ses dérivés à titre de coupleur. Sont également décrites, notamment dans la demande de brevet DE 3 930 446, des compositions pour la teinture des fibres kératiniques renfermant certains dérivés d'indoles, ainsi qu'éventuellement des précurseurs de colorants d'oxydation ou des coupleurs comme des méta-aminophénols, des méta-phénylènediamine, des méta-diphénols ou des pyrazolones.

Or, la demanderesse vient maintenant de découvrir qu'il est possible d'obtenir de nouvelles teintures peu sélectives et particulièrement résistantes, capables d'engendrer des colorations intenses dans des nuances variées, en associant au moins une base d'oxydation, au moins un coupleur indolique convenablement sélectionné et au moins un coupleur hétérocyclique additionnel également convenablement sélectionné, et différent dudit coupleur indolique.

Cette découverte est à la base de la présente invention.

L'invention a donc pour objet une composition pour la teinture d'oxydation des fibres kératiniques et en particulier des fibres kératiniques humaines telles que les cheveux, caractérisée par le fait qu'elle comprend, dans un milieu approprié pour la teinture :
(a) au moins une base d'oxydation,
(b) au moins un coupleur indolique de formule (I) suivante et/ou au moins l'un de ses sels d'addition avec un acide : 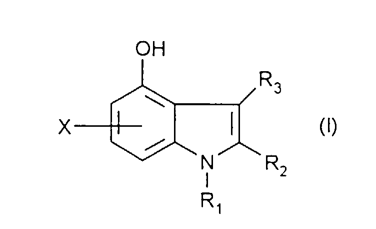 dans laquelle :
   R₁ représente un atome d'hydrogène ou un radical alkyle en C₁-C₄,
   R₂ et R₃, identiques ou différents, représentent un atome d'hydrogène ou un radical alkyle en C₁-C₄, carboxyle ou alcoxy(C₁-C₄)carbonyle,
   X représente un atome d'hydrogène ou d'halogène, un radical alkyle en C₁-C₄, alcoxy en C₁-C₁₈ ou acétylamino ;
(c) au moins un coupleur hétérocyclique additionnel choisi parmi :
   (i) les dérivés indoliques de formule (II) suivante, et leurs sels d'addition avec un acide : 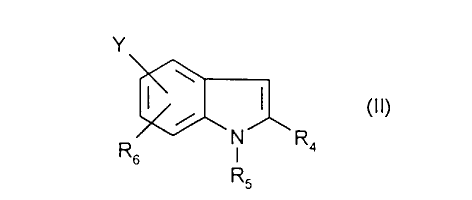 dans laquelle :
      R₄ et R₅, identiques ou différents, représentent un atome d'hydrogène ou un radical alkyle en C₁-C₄ ;
      R₆ représente un atome d'hydrogène, un radical alkyle en C₁-C₄ ou hydroxyle ;
      Y représente un radical hydroxyle ou NHR₇ dans lequel R₇ représente un atome d'hydrogène, un radical alkyle en C₁-C₄ ou hydroxyalkle en C₁-C₄;
      sous réserve que :
      - lorsque R₆ désigne hydroxyle, alors il occupe la position 6, Y désigne hydroxyle et occupe la position 5 et R₄ et R₅ représentent un atome d'hydrogène,
      - lorsque que Y désigne hydroxyle, alors il occupe la position 6 ou 7, et R₆ est différent d'hydroxyle,
      - lorsque Y désigne amino, alors il occupe la position 4, 6 ou 7 ;
   (ii) les dérivés de benzimidazole de formule (III) suivante, et leurs sels d'addition avec un acide : 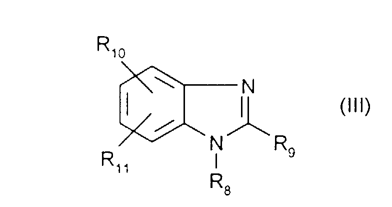 dans laquelle :
      R₈ représente un atome d'hydrogène ou un radical alkyle en C₁-C₄,
      R₉ représente un atome d'hydrogène, un radical alkyle en C₁-C₄ ou phényle,
      R₁₀ représente un radical hydroxyle, amino ou méthoxy,
      R₁₁ représente un atome d'hydrogène, un radical hydroxyle, méthoxy ou alkyle en C₁-C₄;
      sous réserve que :
      - lorsque R₁₀ désigne un radical amino, alors il occupe la position 4,
      - lorsque R₁₀ occupe la position 4, alors R₁₁ occupe la position 7,
      - lorsque R₁₀ occupe la position 5, alors R₁₁ occupe la position 6;
   (iii) les dérivés de benzomorpholine de formule (IV) suivante, et leurs sels d'addition avec un acide : 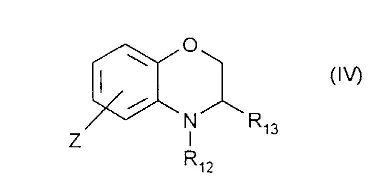 dans laquelle :
      R₁₂ et R₁₃, identiques ou différents, représentent un atome d'hydrogène ou un radical alkyle en C₁-C₄,
      Z représente un radical hydroxyle ou amino ;
   (iv) les dérivés de pyridine de formule (V) suivante, et leurs sels d'addition avec un acide : 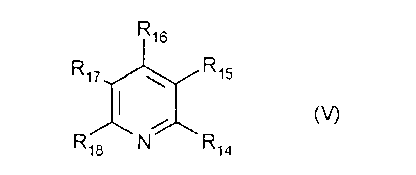 dans laquelle :
      R₁₄ représente un atome d'hydrogène, un radical hydroxyle, alcoxy en C₁-C₄, monohydroxyalcoxy en C₁-C₄, polyhydroxyalcoxy en C₂-C₄, amino ou le groupement -OCH₂CH₂COCH₂CH₂OH,
      R₁₅ et R₁₇, identiques ou différents, représentent un atome d'hydrogène, un radical hydroxyle, amino ou alkyle en C₁-C₄,
      R₁₆ représente un atome d'hydrogène ou un radical alkyle en C₁-C₄,
      R₁₈ représente un atome d'hydrogène, un radical hydroxyle, alcoxy en C₁-C₄, monohydroxyalcoxy en C₁-C₄, polyhydroxyalcoxy en C₂-C₄ ou amino,
      sous réserve que lorsque R₁₄ représente un radical polyhydroxyalcoxy ou le groupement -OCH₂CH₂COCH₂CH₂OH, alors R₁₅ et R₁₇ représentent un radical amino ;
      et étant enfin entendu que ces composés de formule (V) ne comportent pas plus de deux groupements amino (substitués ou non substitués) ou pas plus de deux groupements hydroxyle ou pas plus de un groupement amino et un groupement hydroxyle par molécule, ces groupements amino et/ou hydroxyle étant obligatoirement en position méta l'un par rapport à l'autre ;
   (v) les dérivés d'indoline choisis parmi la 6-hydroxy indoline, la 6-amino indoline, la 5,6-dihydroxyindoline, et leurs sels d'addition avec un acide ;
   (vi) les dérivés de quinoline de formule (VI) suivante, et leurs sels d'addition avec un acide : 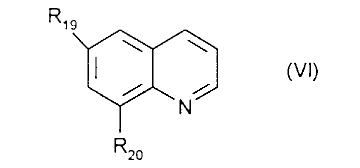 dans laquelle :
      R₁₉ désigne un radical hydroxyle ou alcoxy en C₁-C₄,
      R₂₀ désigne un atome d'hydrogène ou radical amino ;
   (vii) les dérivés de sésamol de formule (VII) suivante, et leurs sels d'addition avec un acide : 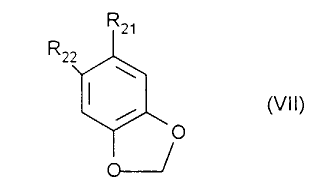 dans laquelle :
      R₂₁ désigne un radical hydroxyle ou amino,
      R₂₂ désigne un atome d'halogène ou radical alcoxy en C₁-C₄.

La composition de teinture d'oxydation conforme à l'invention permet d'obtenir des colorations aux nuances variées, peu sélectives et présentant d'excellentes propriétés de résistance à la fois vis à vis des agents atmosphériques tels que la lumière et les intempéries et vis à vis de la transpiration et des différents traitements que peuvent subir les cheveux (shampooings, déformations permanentes).

L'invention a également pour objet un procédé de teinture d'oxydation des fibres kératiniques mettant en oeuvre cette composition.

La ou les bases d'oxydation utilisables dans le cadre des compositions tinctoriales conformes à l'invention sont de préférence choisies parmi les paraphénylènediamines, les bis-phénylalkylènediamines, les para-aminophénols, les ortho-aminophénols, les bases hétérocycliques, et leurs sels d'addition avec un acide.

Les sels d'addition avec un acide utilisables dans le cadre des compositions tinctoriales de l'invention sont notamment choisis parmi les chlorhydrates, les bromhydrates, les sulfates et les tartrates.

Parmi les paraphénylènediamines utilisables à titre de bases d'oxydation dans le cadre des compositions conformes à l'invention, on peut notamment citer les composés répondant à la formule (VIII) suivante, et leurs sels d'addition avec un acide: 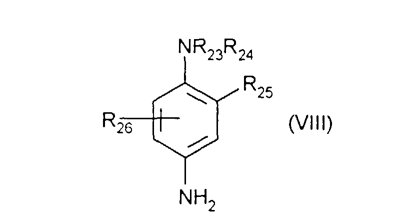 dans laquelle :
R₂₃ représente un atome d'hydrogène, un radical alkyle en C₁-C₄, monohydroxyalkyle en C₁-C₄, polyhydroxyalkyle en C₂-C₄ ou alcoxy(C₁-C₄)alkyle(C₁-C₄),
R₂₄ représente un atome d'hydrogène, un radical alkyle en C₁-C₄, monohydroxyalkyle en C₁-C₄ ou polyhydroxyalkyle en C₂-C₄,
R₂₅ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄,
R₂₆ représente un atome d'hydrogène ou un radical alkyle en C₁-C₄.

Dans la formule (VIII) ci-dessus des paraphénylènediamines, et lorsque R₂₆ est différent d'un atome d'hydrogène, alors R₂₃ et R₂₄ représentent de préférence un atome d'hydrogène et R₂₅ est de préférence identique à R₂₆, et lorsque R₂₅ représente un atome d'halogène, alors R₂₃, R₂₄ et R₂₆ représentent de préférence un atome d'hydrogène.

Parmi les paraphénylènediamines de formule (VIII) ci-dessus, on peut plus particulièrement citer la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la **N,N**-bis-(β-hydroxyéthyl) paraphénylènediamine, le 4-amino 1-(β-méthoxyéthyl)amino benzène, la 2-chloro paraphénylènediamine, et leurs sels d'addition avec un acide.

Parmi les bis-phénylalkylènediamines utilisables à titre de bases d'oxydation dans le cadre des compositions conformes à l'invention, on peut notamment citer les composés répondant à la formule (IX) suivante, et leurs sels d'addition avec un acide : 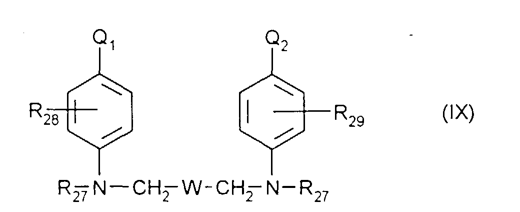 dans laquelle :
Q₁ et Q₂, identiques ou différents, représentent un radical hydroxyle ou NHR₃₀ dans lequel R₃₀ représente un atome d'hydrogène ou un radical alkyle en C₁-C₄,
R₂₇ représente un atome d'hydrogène, un radical alkyle en C₁-C₄, monohydroxyalkyle en C₁-C₄, polyhydroxyalkyle en C₂-C₄ ou aminoalkyle en C₁-C₄ dont le reste amino peut être substitué,
R₂₈ et R₂₉, identiques ou différents, représentent un atome d'hydrogène ou d'halogène ou un radical alkyle en C₁-C₄,
W représente un radical pris dans le groupe constitué par les radicaux suivants : 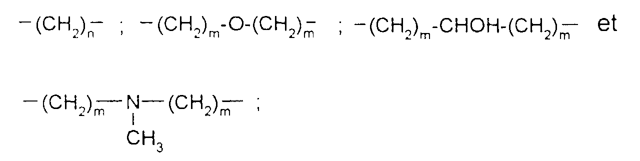 dans lesquels n est un nombre entier compris entre 0 et 8 inclusivement et m est un nombre entier compris entre 0 et 4 inclusivement.

Parmi les bis-phénylalkylènediamines de formules (IX) ci-dessus, on peut plus particulièrement citer le **N,N'**-bis-(β-hydroxyéthyl) **N,N'**-bis-(4'-aminophényl) 1,3-diamino propanol, la **N,N'**-bis-(β-hydroxyéthyl) **N,N'**-bis-(4'-aminophényl) éthylènediamine, la **N,N'**-bis-(4-aminophényl) tétraméthylènediamine, la **N,N'**-bis-(β-hydroxyéthyl) **N,N'**-bis-(4-aminophényl) tétraméthylènediamine, la **N,N'**-bis-(4-méthylaminophényl) tétraméthylènediamine, la **N,N'**-bis-(éthyl) **N,N'**-bis-(4'-amino, 3'-méthylphényl) éthylènediamine, et leurs sels d'addition avec un acide.

Parmi ces bis-phénylalkylènediamines de formule (IX), le **N,N'**-bis-(β-hydroxyéthyl) **N,N'**-bis-(4'-aminophényl) 1,3-diamino propanol ou l'un de ses sels d'addition avec un acide sont particulièrement préférés.

Parmi les para-aminophénols utilisables à titre de bases d'oxydation dans le cadre des compositions conformes à l'invention, on peut notamment citer les composés répondant à la formule (X) suivante, et leurs sels d'addition avec un acide : 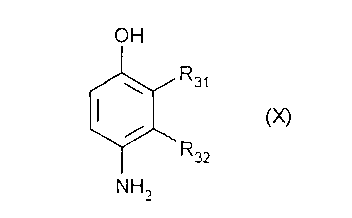 dans laquelle :
R₃₁ représente un atome d'hydrogène, un radical alkyle en C₁-C₄, monohydroxyalkyle en C₁-C₄, alcoxy(C₁-C₄)alkyle(C₁-C₄) ou aminoalkyle en C₁-C₄,
R₃₂ représente un atome d'hydrogène ou de fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₃₁ ou R₃₂ représente un atome d'hydrogène ;

Parmi les para-aminophénols de formule (X) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, et leurs sels d'addition avec un acide.

Parmi les ortho-aminophénols utilisables à titre de bases d'oxydation dans le cadre des compositions conformes à l'invention, on peut notamment citer le 2-amino phénol, le 2-amino 1-hydroxy 5-méthyl benzène, le 2-amino 1-hydroxy 6-méthyl benzène, le 5-acétamido 2-amino phénol, et leurs sels d'addition avec un acide.

Parmi les bases hétérocycliques utilisables à titre de bases d'oxydation dans le cadre des compositions conformes à l'invention, on peut notamment citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diaminopyridine, et leurs sels d'addition avec un acide.
Parmi dérivés pyrimidiniques, on peut plus particulièrement citer les composés décrits par exemple dans les brevets allemand DE 2 359 399 ou japonais JP 88-169 571 et JP 91-333 495, comme la 2,4,5,6-tétra-aminopyrimidine, la 4-hydroxy 2,5,6-triaminopyrimidine, et leurs sels d'addition avec un acide. Parmi les dérivés pyrazoliques, on peut plus particulièrement citer les composés décrits dans les brevets DE 3 843 892, DE 4 133 957 et demandes de brevet WO 94/08969 et WO 94/08970 comme le 4,5-diamino 1-méthyl pyrazole, le 3,4-diamino pyrazole, et leurs sels d'addition avec un acide.
Parmi les coupleurs indoliques de formule (I) ci-dessus, on peut particulièrement citer le 4-hydroxy indole, le 4-hydroxy 5-éthoxy indole, le 4-hydroxy 5-méthoxy indole, le 4-hydroxy 1-méthyl 5-éthoxy indole, le 4-hydroxy 2-éthoxycarbonyl 5-éthoxy indole, le 4-hydroxy 2-méthyl 5-éthoxy indole, le 4-hydroxy 5-méthyl indole, le 4-hydroxy 2-méthyl indole, le 4-hydroxy 1-méthyl indole, et leurs sels d'addition avec un acide.

Parmi les dérivés indoliques de formule (Il) ci-dessus, utilisables à titre de coupleurs hétérocycliques additionnels dans les compositions conformes à l'invention, on peut plus particulièrement citer le 6-hydroxy indole, le 7-amino indole, le 6-amino indole, le 7-hydroxy indole, le 7-éthyl 6-(β-hydroxyéthyl)amino indole, le 4-amino indole, le 6-hydroxy 1-méthyl indole, le 5,6-dihydroxy indole, et leurs sels d'addition avec un acide.

Parmi les dérivés de benzimidazole de formule (III) ci-dessus, utilisables à titre de coupleurs hétérocycliques additionnels dans les compositions conformes à l'invention, on peut plus particulièrement citer le 4-hydroxy benzimidazole, le 4-amino benzimidazole, le 4-hydroxy 7-méthyl benzimidazole, le 4-hydroxy 2-méthyl benzimidazole, le 1-butyl 4-hydroxy benzimidazole, le 4-amino 2-méthyl benzimidazole, le 5,6-dihydroxy benzimidazole, le 5-hydroxy 6-méthoxy benzimidazole, le 4,7-dihydroxy benzimidazole, le 4,7-dihydroxy 1-méthyl benzimidazole, le 4,7-diméthoxy benzimidazole, le 5,6-dihydroxy 1-méthyl benzimidazole, le 5,6-dihydroxy 2-méthyl benzimidazole, le 5,6-diméthoxy benzimidazole, et leurs sels d'addition avec un acide.

Parmi les dérivés de benzomorpholine de formule (IV) ci-dessus, utilisables à titre de coupleurs hétérocycliques additionnels dans les compositions conformes à l'invention, on peut plus particulièrement citer la 6-hydroxy benzomorpholine, la N-méthyl 6-hydroxy benzomorpholine, la 6-amino benzomorpholine, et leurs sels d'addition avec un acide.

Parmi les dérivés de pyridine de formule (V) ci-dessus, utilisables à titre de coupleurs hétérocycliques additionnels dans les compositions conformes à l'invention, on peut plus particulièrement citer la 2,6-dihydroxy 4-méthyl pyridine, la 2,6-dihydroxy 3,4-diméthyl pyridine, la 3,5-diamino 2,6-diméthoxy pyridine, la 2,6-bis-(β-hydroxyéthyl)oxy 3,5-diamino pyridine, la 3-amino 2,6-diméthoxy 5-hydroxy pyridine, la 2,6-diamino pyridine, le 3-oxo 5-(3',5'-diamino 2'-pyridiloxy) pentanol, le 3-(3',5'-diamino 2'-pyridyloxy) 2-hydroxy propanol, et leurs sels d'addition avec un acide.

Parmi les dérivés de quinoline de formule (VI) ci-dessus, utilisables à titre de coupleurs hétérocycliques additionnels dans les compositions conformes à l'invention, on peut plus particulièrement citer la 6-hydroxy quinoline, la 8-amino 6-méthoxy quinoline, et leurs sels d'addition avec un acide.

Parmi les dérivés de sésamol de formule (VII) ci-dessus, utilisables à titre de coupleurs hétérocycliques additionnels dans les compositions conformes à l'invention, on peut plus particulièrement citer le 2-bromo 4,5-méthylènedioxy phénol, le 1-amino 6-méthoxy 3,4-méthylènedioxy benzène, et leurs sels d'addition avec un acide.

Selon une forme de réalisation particulièrement préférée de l'invention, les compositions de teinture d'oxydation conformes à l'invention renferment au moins une des associations ternaires suivantes :
(a) : paraphénylènediamine,
(b) : 4-hydroxy indole,
(c) : 6-hydroxy indole ;

(a) : paraphénylènediamine,
(b) : 4-hydroxy indole,
(c) : 4-hydroxy benzimidazole ;

(a) : paraphénylènediamine,
(b) : 4-hydroxy indole,
(c) : 6-hydroxy benzomorpholine;

(a) : para-aminophénol,
(b) : 4-hydroxy indole,
(c) : 6-hydroxy indole ;

(a) : 4-amino 3-méthyl phénol,
(b) : 4-hydroxy indole,
(c) : 6-hydroxy indole ;

(a) : 2-amino phénol,
(b) : 4-hydroxy indole,
(c) : 6-hydroxy indole ;

(a): 4,5-diamino 1-méthyl pyrazole,
(b) : 4-hydroxy indole,
(c) : 6-hydroxy indole ;

(a) : 4,5-diamino 1-méthyl pyrazole,
(b) : 4-hydroxy indole,
(c) : 4-hydroxy benzimidazole ;

(a) : 4,5-diamino 1-méthyl pyrazole,
(b) : 4-hydroxy indole,
(c) : 6-hydroxy benzomorpholine;

(a) : 2,6-diméthyl paraphénylènediamine,
(b) : 4-hydroxy indole,
(c) : 6-hydroxy indole ;

(a) : 2,6-diméthyl paraphénylènediamine,
(b) : 4-hydroxy indole,
(c) : 4- hydroxy benzimidazole ;

(a) : 2,6-diméthyl paraphénylènediamine,
(b) : 4-hydroxy indole,
(c) : 6-hydroxy benzomorpholine;

(a) : 2-β-hydroxyéthyl paraphénylènediamine,
(b) : 4-hydroxy indole,
(c) : 6-hydroxy indole ;

(a) : 2-β-hydroxyéthyl paraphénylènediamine,
(b) : 4-hydroxy indole,
(c) : 4- hydroxy benzimidazole ;

(a) : paraphénylènediamine,
(b) : 4-hydroxy 1-N-méthyl indole,
(c) : 6-hydroxy indole ;

(a) : paraphénylènediamine
(b) : 4-hydroxy 2-méthyl indole,
(c) : 6-hydroxy indole ;

(a) : para-aminophénol,
(b) : 4-hydroxy 1-N-méthyl indole,
(c) : 6-hydroxy indole ;

(a) : para-aminophénol,
(b) : 4-hydroxy 2-méthyl indole,
(c) : 6-hydroxy indole ;

(a) : 4-amino 3-méthyl phénol,
(b) : 4-hydroxy 1-N-méthyl indole,
(c) : 6-hydroxy indole ;

(a) : 4-amino 3-méthyl phénol,
(b) : 4-hydroxy 2-méthyl indole,
(c) : 6-hydroxy indole ;

a) : paratoluylènediamine,
(b) : 4-hydroxy indole,
(c) : 6-hydroxy indole ;

(a) : paratoluylènediamine,
(b) : 4-hydroxy indole,
(c) : 4-hydroxy benzimidazole ;

(a) : paratoluylènediamine,
(b) : 4-hydroxy indole,
(c) : 6-hydroxy benzomorpholine.

(a) : 4-amino 3-méthyl phénol,
(b) : 4-hydroxy indole,
(c) : 6-hydroxy benzomorpholine;

(a) : 2,6-diméthyl paraphénylènediamine,
(b) : 4-hydroxy 1-méthyl indole,
(c) : 1-amino 6-méthoxy 3,4-méthylènedioxy benzène ;

(a) : 3,4-diamino pyrazole,
(b) : 4-hydroxy indole,
(c) : 2,6-diamino pyridine.

L'ensemble des bases d'oxydation conformes à l'invention représente de préférence de 0,0005 à 12 % en poids environ du poids total de la composition tinctoriale, et encore plus préférentiellement de 0,005 à 6 % en poids environ de ce poids.

Le ou les coupleurs indoliques de formule (I) conformes à l'invention représentent de préférence de 0,0001 à 5 % en poids environ du poids total de la composition tinctoriale, et encore plus préférentiellement de 0,005 à 3 % en poids environ de ce poids.

Le ou les coupleurs hétérocycliques additionnels conformes à l'invention représentent de préférence de 0,0001 à 10 % en poids environ du poids total de la composition tinctoriale, et encore plus préférentiellement de 0,005 à 5 % en poids environ de ce poids.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁ -C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ et encore plus préférentiellement entre 5 et 11 environ. Il peut être ajusté à la valeur désirée au moyen d'agents acidifiants ou alcalinisants habituellement utilisés en teinture des fibres kératiniques.

Parmi les agents acidifiants on peut citer, à titre d'exemple, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (XI) suivante : 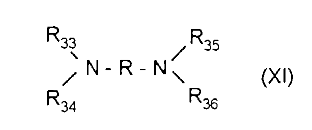 dans laquelle R est un reste propylène éventuellement substitué par un groupement hydroxyle ou un radical alkyle en C₁-C₄; R₃₃, R₃₄, R₃₅ et R₃₆, identiques ou différents, représentent un atome d'hydrogène, un radical alkyle en C₁-C₄ ou hydroxyalkyle en C₁-C₄.

La composition tinctoriale conforme à l'invention peut encore contenir, en plus des colorants définis ci-dessus, d'autres bases d'oxydation et/ou d'autres coupleurs et/ou des colorants directs, notamment pour modifier les nuances ou les enrichir en reflets.

La composition tinctoriale selon l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir ce ou ces éventuels composés complémentaires de manière telle que les propriétés avantageuses attachées intrinsèquement à l'association ternaire conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres la composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention.

### EXEMPLES

### EXEMPLES 1 à 5 DE TEINTURE EN MILIEU ALCALIN

On a réalisé les compositions tinctoriales suivantes (teneurs en grammes):

| **EXEMPLE** | **1** | **2** | **3** | **4** | **5** |
|---|---|---|---|---|---|
| Para-aminophénol | 0,2 | | | | |
| Dichlorhydrate de 2-(β-hydroxyéthyl) para phénylènediamine | | 0,3 | | | |
| 4-amino 3-méthyl phénol | | | 0,3 | | |
| Dichlorhydrate de 2,6-diméthyl para phénylènediamine | | | | 1,0 | |
| Dichlorhydrate de 3,4-diamino pyrazole | | | | | 0,3 |
| 4-hydroxy indole | 0,1 | 0,2 | 0,25 | | 0,05 |
| 4-hydroxy 1-méthyl indole | | | | 0,5 | |
| 6-hydroxy indole | 0,1 | | | | |
| Bromhydrate de 4-hydroxy benzimidazole | | 0,1 | | | |
| 6-hydroxy benzomorpholine | | | 0,05 | | |
| Chlorhydrate de 1-amino 6-méthoxy 3,4-méthylènedioxy benzène | | | | 0,5 | |
| 2,6-diamino pyridine | | | | | 0,25 |
| Support de teinture commun | (*) | (*) | (*) | (*) | (*) |
| Eau déminéralisée q.s.p. | 100 g | 100 g | 100 g | 100 g | 100 g |

(*) : Support de teinture commun :
- Alcool oléique polyglycérolé à 2 moles de glycérol 4,0 g
- Alcool oléique polyglycérolé à 4 moles de glycérol, à 78 % de matières actives (M.A.) 5,69 g M.A.
- Acide oléique 3,0 g
- Amine oléique à 2 moles d'oxyde d'éthylène vendue sous la dénomination commerciale ETHOMEEN O12 par la société AKZO 7,0 g
- Laurylamino succinamate de diéthylaminopropyle, sel de sodium, à 55 % de M.A. 3,0 g M.A.
- Alcool oléique 5,0 g
- Diéthanolamide d'acide oléique 12,0 g
- Propylèneglycol 3,5 g
- Alcool éthylique 7,0 g
- Dipropylèneglycol 0,5 g
- Monométhyléther de propylèneglycol 9,0 g
- Métabisulfite de sodium en solution aqueuse, à 35 % de M.A. 0,455 g M.A.
- Acétate d'ammonium 0,8 g
- Antioxydant, séquestrant q.s.
- Parfum, conservateur q.s.
- Ammoniaque à 20 % de NH₃ 10,0 g

Au moment de l'emploi, on a mélangé chaque composition tinctoriale avec une quantité égale en poids d'une solution d'eau oxygénée à 20 volumes (6 % en poids).

Chaque composition résultante présentait un pH d'environ 10,2 et a été appliquée pendant 30 minutes sur des mèches de cheveux gris à 90 % de blancs, naturels ou permanentés. Les mèches de cheveux ont ensuite été rincées, lavées avec un shampooing standard puis séchées.

Les mèches de cheveux ont été teintes dans les nuances figurant dans le tableau ci-dessous :

| **EXEMPLE** | **NUANCE SUR CHEVEUX NATURELS** | **NUANCE SUR CHEVEUX PERMANENTES** |
|---|---|---|
| **1** | beige cuivré irisé léger | irisé légèrement cuivré |
| **2** | cendré bleuté léger | cendré bleuté puissant |
| **3** | irisé léger | irisé |
| **4** | blond foncé cendré mat | châtain naturel mat |
| **5** | rose fuchsia puissant | rose fuchsia très puissant |

### EXEMPLE 6 DE TEINTURE EN MILIEU ACIDE

On a réalisé la composition tinctoriale suivante (teneurs en grammes):

| **EXEMPLE** | **6** |
|---|---|
| Dichlorhydrate de 2,6-diméthyl paraphénylènediamine | 0,5 |
| 4-hydroxy indole | 0,3 |
| 6-hydroxy indole | 0,2 |
| Support de teinture | (**) |
| Eau déminéralisée q.s.p. | 100 g |

(**) : Support de teinture :
- Alcool oléique polyglycérolé à 2 moles de glycérol 4,0 g
- Alcool oléique polyglycérolé à 4 moles de glycérol, à 78 % de matières actives (M.A.) 5,69 g M.A.
- Acide oléique 3,0 g
- Amine oléique à 2 moles d'oxyde d'éthylène vendue sous la dénomination commerciale ETHOMEEN O12 par la société AKZO 7,0 g
- Laurylamino succinamate de diéthylaminopropyle, sel de sodium, à 55 % de M.A. 3,0 g M.A.
- Alcool oléique 5,0 g
- Diéthanolamide d'acide oléique 12,0 g
- Propylèneglycol 3,5 g
- Alcool éthylique 7,0 g
- Dipropylèneglycol 0,5 g
- Monométhyléther de propylèneglycol 9,0 g
- Métabisulfite de sodium en solution aqueuse, à 35 % de M.A. 0,455 g M.A.
- Acétate d'ammonium 0,8 g
- Antioxydant, séquestrant q.s.
- Parfum, conservateur q.s.
- Monoéthanolamine q.s.p. pH 9,8

Au moment de l'emploi, on a mélangé la composition tinctoriale avec une quantité égale en poids d'une solution d'eau oxygénée à 20 volumes (6 % en poids), et dont le pH avait été ajusté entre 1 et 1,5 par 2,5 g d'acide orthophosphorique pour 100 g d'eau oxygénée.

La composition résultante présentait un pH de 6,6 et a été appliquée pendant 30 minutes sur des mèches de cheveux gris à 90 % de blancs, naturels ou permanentés. Les mèches de cheveux ont ensuite été rincées, lavées avec un shampooing standard puis séchées.

Les mèches de cheveux ont été teintes dans les nuances figurant dans le tableau ci-dessous :

| **EXEMPLE** | **NUANCE SUR CHEVEUX NATURELS** | **NUANCE SUR CHEVEUX PERMANENTES** |
|---|---|---|
| **6** | blond foncé cendré irisé | châtain clair cendré violacé |

## Revendications

1. Composition pour la teinture d'oxydation des fibres kératiniques et en particulier des fibres kératiniques humaines telles que les cheveux, caractérisée par le fait qu'elle comprend, dans un milieu approprié pour la teinture :
(a) au moins une base d'oxydation,
(b) au moins un coupleur indolique de formule (I) suivante et/ou au moins l'un de ses sels d'addition avec un acide : 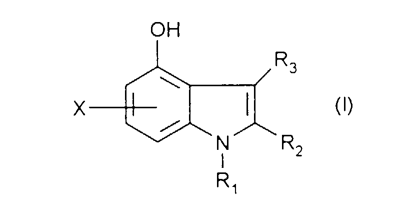 dans laquelle :
R₁ représente un atome d'hydrogène ou un radical alkyle en C₁-C₄,
R₂ et R₃ , identiques ou différents, représentent un atome d'hydrogène ou un radical alkyle en C₁-C₄, carboxyle ou alcoxy(C₁-C₄)carbonyle,
X représente un atome d'hydrogène ou d'halogène, un radical alkyle en C₁-C₄, alcoxy en C₁-C₁₈ ou acétylamino ;
(c) au moins un coupleur hétérocyclique additionnel choisi parmi :
(i) les dérivés indoliques de formule (Il) suivante, et leurs sels d'addition avec un acide : 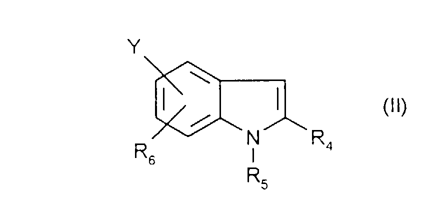 dans laquelle :
R₄ et R₅, identiques ou différents, représentent un atome d'hydrogène ou un radical alkyle en C₁-C₄;
R₆ représente un atome d'hydrogène, un radical alkyle en C₁-C₄ ou hydroxyle ;
Y représente un radical hydroxyle ou NHR₇ dans lequel R₇ représente un atome d'hydrogène, un radical alkyle en C₁-C₄ ou hydroxyalkyle en C₁-C₄ ;
sous réserve que :
- lorsque R₆ désigne hydroxyle, alors il occupe la position 6, Y désigne hydroxyle et occupe la position 5 et R₄ et R₅ représentent un atome d'hydrogène,
- lorsque que Y désigne hydroxyle, alors il occupe la position 6 ou 7, et R₆ est différent d'hydroxyle,
- lorsque Y désigne amino, alors il occupe la position 4, 6 ou 7 ;
(ii) les dérivés de benzimidazole de formule (III) suivante, et leurs sels d'addition avec un acide : 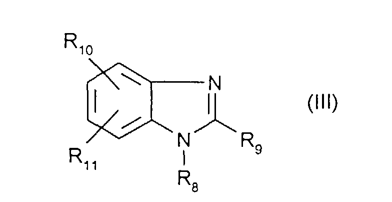 dans laquelle :
R₈ représente un atome d'hydrogène ou un radical alkyle en C₁-C₄,
R₉ représente un atome d'hydrogène, un radical alkyle en C₁-C₄ ou phényle,
R₁₀ représente un radical hydroxyle, amino ou méthoxy,
R₁₁ représente un atome d'hydrogène, un radical hydroxyle, méthoxy ou alkyle en C₁-C₄;
sous réserve que :
- lorsque R₁₀ désigne un radical amino, alors il occupe la position 4,
- lorsque R₁₀ occupe la position 4, alors R₁₁ occupe la position 7,
- lorsque R₁₀ occupe la position 5, alors R₁₁ occupe la position 6;
(iii) les dérivés de benzomorpholine de formule (IV) suivante, et leurs sels d'addition avec un acide : 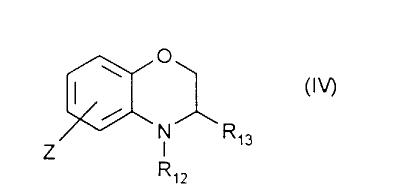 dans laquelle :
R₁₂ et R₁₃, identiques ou différents, représentent un atome d'hydrogène ou un radical alkyle en C₁-C₄,
Z représente un radical hydroxyle ou amino ;
(iv) les dérivés de pyridine de formule (V) suivante, et leurs sels d'addition avec un acide : 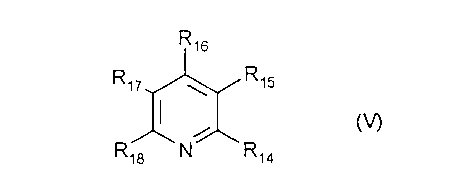 dans laquelle :
R₁₄ représente un atome d'hydrogène, un radical hydroxyle, alcoxy en C₁-C₄, monohydroxyalcoxy en C₁-C₄, polyhydroxyalcoxy en C₂-C₄, amino ou le groupement -OCH₂CH₂COCH₂CH₂OH,
R₁₅ et R₁₇, identiques ou différents, représentent un atome d'hydrogène, un radical hydroxyle, amino ou alkyle en C₁-C₄,
R₁₆ représente un atome d'hydrogène ou un radical alkyle en C₁-C₄,
R₁₈ représente un atome d'hydrogène, un radical hydroxyle, alcoxy en C₁-C₄, monohydroxyalcoxy en C₁-C₄, polyhydroxyalcoxy en C₂-C₄ ou amino,
sous réserve que lorsque R₁₄ représente un radical polyhydroxyalcoxy ou le groupement -OCH₂CH₂COCH₂CH₂OH, alors R₁₅ et R₁₇ représentent un radical amino ;
et étant enfin entendu que ces composés de formule (V) ne comportent pas plus de deux groupements amino (substitués ou non substitués) ou pas plus de deux groupements hydroxyle ou pas plus de un groupement amino et un groupement hydroxyle par molécule, ces groupements amino et/ou hydroxyle étant obligatoirement en position méta l'un par rapport à l'autre ;
(v) les dérivés d'indoline choisis parmi la 6-hydroxy indoline, la 6-amino indoline, la 5,6-dihydroxyindoline, et leurs sels d'addition avec un acide ;
(vi) les dérivés de quinoline de formule (VI) suivante, et leurs sels d'addition avec un acide : 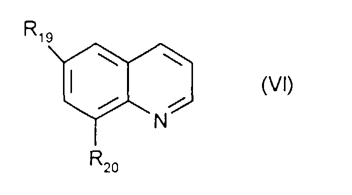 dans laquelle :
R₁₉ désigne un radical hydroxyle ou alcoxy en C₁-C₄,
R₂₀ désigne un atome d'hydrogène ou radical amino ;
(vii) les dérivés de sésamol de formule (VII) suivante, et leurs sels d'addition avec un acide : 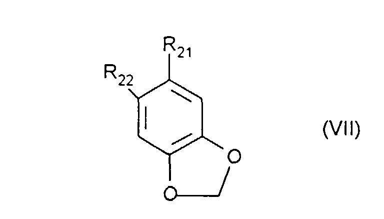 dans laquelle :
R₂₁ désigne un radical hydroxyle ou amino,
R₂₂ désigne un atome d'halogène ou radical alcoxy en C₁-C₄.

2. Composition selon la revendication 1, caractérisée par le fait que la ou les bases d'oxydation sont choisies parmi les paraphénylènediamines, les bis-phénylalkylènediamines, les para-aminophénols, les ortho-aminophénols, les bases hétérocycliques, et leurs sels d'addition avec un acide.

3. Composition selon la revendication 2, caractérisée par le fait que les paraphénylènediamines sont choisies parmi les composés répondant à la formule (VIII) suivante, et leurs sels d'addition avec un acide ; 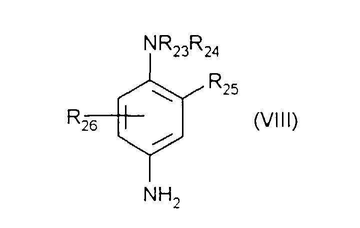 dans laquelle :
R₂₃ représente un atome d'hydrogène, un radical alkyle en C₁-C₄, monohydroxyalkyle en C₁-C₄, polyhydroxyalkyle en C₂-C₄ ou alcoxy(C₁-C₄)alkyle(C₁-C₄),
R₂₄ représente un atome d'hydrogène, un radical alkyle en C₁-C₄, monohydroxyalkyle en C₁-C₄ ou polyhydroxyalkyle en C₂-C₄,
R₂₃ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄,
R₂₆ représente un atome d'hydrogène ou un radical alkyle en C₁-C₄.

4. Composition selon la revendication 3, caractérisée par le fait que les paraphénylènediamines de formule (VIII) sont choisies parmi la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, le 4-amino 1-(β-méthoxyéthyl)amino benzène, la 2-chloro paraphénylènediamine, et leurs sels d'addition avec un acide.

5. Composition selon la revendication 2, caractérisée par le fait que les bis-phénylalkylènediamines sont choisies parmi les composés répondant à la formule (IX) suivante, et leurs sels d'addition avec un acide : 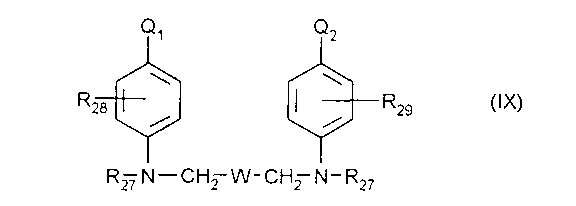 dans laquelle :
Q₁ et Q₂, identiques ou différents, représentent un radical hydroxyle ou NHR₃₀ dans lequel R₃₀ représente un atome d'hydrogène ou un radical alkyle en C₁-C₄,
R₂₇ représente un atome d'hydrogène, un radical alkyle en C₁-C₄, monohydroxyalkyle en C₁-C₄, polyhydroxyalkyle en C₂-C₄ ou aminoalkyle en C₁-C₄ dont le reste amino peut être substitué,
R₂₈ et R₂₉, identiques ou différents, représentent un atome d'hydrogène ou d'halogène ou un radical alkyle en C₁-C₄,
W représente un radical pris dans le groupe constitué par les radicaux suivants : 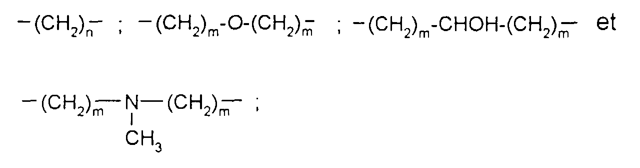 dans lesquels n est un nombre entier compris entre 0 et 8 inclusivement et m est un nombre entier compris entre 0 et 4 inclusivement.

6. Composition selon la revendication 5, caractérisée par le fait que les bis-phénylalkylènediamines de formules (IX) sont choisies parmi le **N,N'**-bis-(β-hydroxyéthyl) **N,N'**-bis-(4'-aminophényl) 1,3-diamino propanol, la **N,N'**-bis-(β-hydroxyéthyl) **N,N'**-bis-(4'-aminophényl) éthylènediamine, la **N,N'**-bis-(4-aminophényl) tétraméthylènediamine, la **N,N'**-bis-(β-hydroxyéthyl) **N,N'**-bis-(4-aminophényl) tétraméthylènediamine, la **N,N'**-bis-(4-méthylamino phényl) tétraméthylènediamine, la **N,N'**-bis-(éthyl) **N,N'**-bis-(4'-amino, 3'-méthylphényl) éthylènediamine, et leurs sels d'addition avec un acide.

7. Composition selon la revendication 2, caractérisée par le fait que les para-aminophénols sont choisis parmi les composés répondant à la formule (X) suivante, et leurs sels d'addition avec un acide : 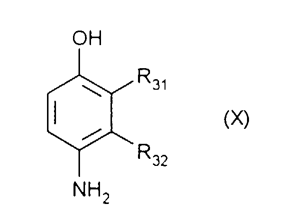 dans laquelle :
R₃₁ représente un atome d'hydrogène, un radical alkyle en C₁-C₄, monohydroxyalkyle en C₁-C₄, alcoxy(C₁-C₄)alkyle(C₁-C₄) ou aminoalkyle en C₁-C₄,
R₃₂ représente un atome d'hydrogène ou de fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₃₁ ou R₃₂ représente un atome d'hydrogène ;

8. Composition selon la revendication 7, caractérisée par le fait que les para-aminophénols de formule (X) sont choisis parmi le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, et leurs sels d'addition avec un acide.

9. Composition selon la revendication 2, caractérisée par le fait que les ortho-aminophénols sont choisis parmi le 2-amino phénol, le 2-amino 1-hydroxy 5-méthyl benzène, le 2-amino 1-hydroxy 6-méthyl benzène, le 5-acétamido 2-amino phénol, et leurs sels d'addition avec un acide.

10. Composition selon la revendication 2, caractérisée par le fait que les bases hétérocycliques sont choisies parmi les dérivés pyridiniques, les dérivés pyrimidiniques, les dérivés pyrazoliques, et leurs sels d'addition avec un acide.

11. Composition selon la revendication 10, caractérisée par le fait que les bases d'oxydation hétérocycliques sont choisies parmi la 2,5-diaminopyridine, la 2,4,5,6-tétra-aminopyrimidine, la 4-hydroxy 2,5,6-triaminopyrimidine, le 4,5-diamino 1-méthyl pyrazole, le 3,4-diamino pyrazole, et leurs sels d'addition avec un acide.

12. Composition selon l'une quelconque des revendications précédentes, caractérisée par le fait que les coupleurs indoliques de formule (I) sont choisis parmi le 4-hydroxy indole, le 4-hydroxy 5-éthoxy indole, le 4-hydroxy 5-méthoxy indole, le 4-hydroxy 1-méthyl 5-éthoxy indole, le 4-hydroxy 2-éthoxycarbonyl 5-éthoxy indole, le 4-hydroxy 2-méthyl 5-éthoxy indole, le 4-hydroxy 5-méthyl indole, le 4-hydroxy 2-méthyl indole, le 4-hydroxy 1-méthyl indole, et leurs sels d'addition avec un acide.

13. Composition selon l'une quelconque des revendications précédentes, caractérisée par le fait que les dérivés indoliques de formule (II), utilisés à titre de coupleurs hétérocycliques additionnels, sont choisis parmi le 6-hydroxy indole, le 7-amino indole, le 6-amino indole, le 7-hydroxy indole, le 7-éthyl 6-(β-hydroxyéthyl)amino indole, le 4-amino indole, le 6-hydroxy 1-méthyl indole, le 5,6-dihydroxy indole, et leurs sels d'addition avec un acide.

14. Composition selon l'une quelconque des revendications 1 à 12, caractérisée par le fait que les dérivés de benzimidazole de formule (III), utilisés à titre de coupleurs hétérocycliques additionnels, sont choisis parmi le 4-hydroxy benzimidazole, le 4-amino benzimidazole, le 4-hydroxy 7-méthyl benzimidazole, le 4-hydroxy 2-méthyl benzimidazole, le 1-butyl 4-hydroxy benzimidazole, le 4-amino 2-méthyl benzimidazole, le 5,6-dihydroxy benzimidazole, le 5-hydroxy 6-méthoxy benzimidazole, le 4,7-dihydroxy benzimidazole, le 4,7-dihydroxy 1-méthyl benzimidazole, le 4,7-diméthoxy benzimidazole, le 5,6-dihydroxy 1-méthyl benzimidazole, le 5,6-dihydroxy 2-méthyl benzimidazole, le 5,6-diméthoxy benzimidazole, et leurs sels d'addition avec un acide.

15. Composition selon l'une quelconque des revendications 1 à 12, caractérisée par le fait que les dérivés de benzomorpholine de formule (IV), utilisés à titre de coupleurs hétérocycliques additionnels, sont choisis parmi la 6-hydroxy benzomorpholine, la N-méthyl 6-hydroxy benzomorpholine, la 6-amino benzomorpholine, et leurs sels d'addition avec un acide.

16. Composition selon l'une quelconque des revendications 1 à 12, caractérisée par le fait que les dérivés de pyridine de formule (V), utilisés à titre de coupleurs hétérocycliques additionnels, sont choisis parmi la 2,6-dihydroxy 4-méthyl pyridine, la 2,6-dihydroxy 3,4-diméthyl pyridine, la 3,5-diamino 2,6-diméthoxy pyridine, la 2,6-bis-(β-hydroxyéthyl)oxy 3,5-diamino pyridine, la 3-amino 2,6-diméthoxy 5-hydroxy pyridine, la 2,6-diamino pyridine, le 3-oxo 5-(3',5'-diamino 2'-pyridiloxy) pentanol, le 3-(3',5'-diamino 2'-pyridyloxy) 2-hydroxy propanol, et leurs sels d'addition avec un acide.

17. Composition selon l'une quelconque des revendications 1 à 12, caractérisée par le fait que les dérivés de quinoline de formule (VI), utilisés à titre de coupleurs hétérocycliques additionnels, sont choisis parmi la 6-hydroxy quinoline, la 8-amino 6-méthoxy quinoline, et leurs sels d'addition avec un acide.

18. Composition selon l'une quelconque des revendications 1 à 12, caractérisée par le fait que les dérivés de sésamol de formule (VII), utilisés à titre de coupleurs hétérocycliques additionnels, sont choisis parmi le 2-bromo 4,5-méthylènedioxy phénol, le 1-amino 6-méthoxy 3,4-méthylènedioxy benzène, et leurs sels d'addition avec un acide.

19. Composition selon l'une quelconque des revendications précédentes, caractérisée par le fait que les sels d'addition avec un acide sont choisis parmi les chlorhydrates, les bromhydrates, les sulfates et les tartrates.

20. Composition selon l'une quelconque des revendications précédentes, caractérisée par le fait qu'elle renferme au moins une des associations ternaires suivantes :
(a) : paraphénylènediamine,
(b) : 4-hydroxy indole,
(c) : 6-hydroxy indole ;
(a) : paraphénylènediamine,
(b) : 4-hydroxy indole,
(c) : 4-hydroxy benzimidazole ;
(a) : paraphénylènediamine,
(b) : 4-hydroxy indole,
(c) : 6-hydroxy benzomorpholine;
(a) : para-aminophénol,
(b) : 4-hydroxy indole,
(c) : 6-hydroxy indole ;
(a) : 4-amino 3-méthyl phénol,
(b) : 4-hydroxy indole,
(c) : 6-hydroxy indole ;
(a) : 2-amino phénol,
(b) : 4-hydroxy indole,
(c) : 6-hydroxy indole ;
(a) : 4,5-diamino 1-méthyl pyrazole,
(b) : 4-hydroxy indole,
(c) : 6-hydroxy indole ;
(a) : 4,5-diamino 1-méthyl pyrazole,
(b) : 4-hydroxy indole,
(c) : 4-hydroxy benzimidazole ;
(a) : 4,5-diamino 1-méthyl pyrazole,
(b) : 4-hydroxy indole,
(c) : 6-hydroxy benzomorpholine;
(a) : 2,6-diméthyl paraphénylènediamine,
(b) : 4-hydroxy indole,
(c) : 6-hydroxy indole ;
(a) : 2,6-diméthyl paraphénylènediamine,
(b) : 4-hydroxy indole,
(c) : 4- hydroxy benzimidazole ;
(a) : 2,6-diméthyl paraphénylènediamine,
(b) : 4-hydroxy indole,
(c) : 6-hydroxy benzomorpholine;
(a) : 2-β-hydroxyéthyl paraphénylènediamine,
(b) : 4-hydroxy indole,
(c) : 6-hydroxy indole ;
(a) : 2-β-hydroxyéthyl paraphénylènediamine,
(b) : 4-hydroxy indole,
(c) : 4- hydroxy benzimidazole ;
(a) : paraphénylènediamine,
(b) : 4-hydroxy 1-N-méthyl indole,
(c) : 6-hydroxy indole ;
(a) : paraphénylènediamine
(b) : 4-hydroxy 2-méthyl indole,
(c) : 6-hydroxy indole ;
(a) : para-aminophénol,
(b) : 4-hydroxy 1-N-méthyl indole,
(c) : 6-hydroxy indole ;
(a) : para-aminophénol,
(b) : 4-hydroxy 2-méthyl indole,
(c): 6-hydroxy indole ;
(a) : 4-amino 3-méthyl phénol,
(b) : 4-hydroxy 1-N-méthyl indole,
(c) : 6-hydroxy indole ;
(a) : 4-amino 3-méthyl phénol,
(b) : 4-hydroxy 2-méthyl indole,
(c) : 6-hydroxy indole ;
(a) : paratoluylènediamine,
(b) : 4-hydroxy indole,
(c) : 6-hydroxy indole ;
(a) : paratoluylènediamine,
(b) : 4-hydroxy indole,
(c) : 4-hydroxy benzimidazole ;
(a) : paratoluylènediamine,
(b) : 4-hydroxy indole,
(c) : 6-hydroxy benzomorpholine ;
(a) : 4-amino 3-méthyl phénol,
(b) : 4-hydroxy indole,
(c) : 6-hydroxy benzomorpholine;
(a) : 2,6-diméthyl paraphénylènediamine,
(b) : 4-hydroxy 1-méthyl indole,
(c) : 1-amino 6-méthoxy 3,4-méthylènedioxy benzène ;
(a) : 3,4-diamino pyrazole,
(b) : 4-hydroxy indole,
(c) : 2,6-diamino pyridine.

21. Composition selon l'une quelconque des revendications précédentes, caractérisée par le fait que l'ensemble des bases d'oxydation représente de 0,0005 à 12 % en poids du poids total de la composition tinctoriale.

22. Composition selon la revendication 21, caractérisée par le fait que l'ensemble des bases d'oxydation représente de 0,005 à 6 % en poids du poids total de la composition tinctoriale.

23. Composition selon l'une quelconque des revendications précédentes, caractérisée par le fait que le ou les coupleurs indoliques de formule (I) représentent de 0,0001 à 5 % en poids du poids total de la composition tinctoriale.

24. Composition selon la revendication 23, caractérisée par le fait que le ou les coupleurs indoliques de formule (I) représentent de 0,005 à 3 % en poids du poids total de la composition tinctoriale.

25. Composition selon l'une quelconque des revendications précédentes, caractérisée par le fait que le ou les coupleurs hétérocycliques additionnels représentent de 0,0001 à 10 % en poids du poids total de la composition tinctoriale.

26. Composition selon la revendication 25, caractérisée par le fait que le ou les coupleurs hétérocycliques additionnels représentent de 0,005 à 5 % en poids du poids total de la composition tinctoriale.

27. Composition selon l'une quelconque des revendications précédentes, caractérisée par le fait que le milieu approprié pour la teinture (ou support) est constitué par de l'eau ou par un mélange d'eau et d'au moins un solvant organique choisi parmi les alcanols inférieurs en C₁-C₄, le glycérol, les glycols et éthers de glycols, les alcools aromatiques, les produits analogues et leurs mélanges.

28. Composition selon l'une quelconque des revendications précédentes, caractérisée par le fait qu'elle présente un pH compris entre 3 et 12.

29. Procédé de teinture des fibres kératiniques et en particulier des fibres kératiniques humaines telles que les cheveux caractérisé par le fait que l'on applique sur ces fibres une composition tinctoriale telle que définie à l'une quelconque des revendications 1 à 28 et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

30. Procédé selon la revendication 29, caractérisé par le fait que l'agent oxydant est choisi parmi le peroxyde d'hydrogène, le peroxyde d'urée, les bromates de métaux alcalins, les persels tels que les perborates et persulfates.

31. Dispositif à plusieurs compartiments, ou "kit" de teinture à plusieurs compartiments, dont un premier compartiment renferme une composition tinctoriale telle que définie à l'une quelconque des revendications 1 à 28 et un second compartiment renferme une composition oxydante.

## Patentansprüche

1. Zusammensetzung zum oxidativen Färben von Keratinfasern und insbesondere menschlichen Keratinfasern, wie dem Haar,
dadurch gekennzeichnet, daß
sie in einem zum Färben geeigneten Medium enthält:
(a) mindestens eine Oxidationsbase,
(b) mindestens einen Indolkuppler der folgenden Formel (I) und/oder mindestens ein Additionssalz dieser Verbindung mit einer Säure: 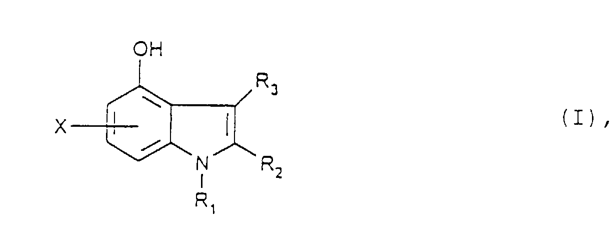 worin bedeuten:
R₁ Wasserstoff oder C₁₋₄-Alkyl,
R₂ und R₃, die identisch oder voneinander verschieden sind, Wasserstoff, C₁₋₄-Alkyl, Carboxy oder C₁₋₄-Alkoxycarbonyl, und
X Wasserstoff, Halogen, C₁₋₄-Alkyl, C₁₋₁₈-Alkoxy oder Acetylamino;
(c) mindestens einen zusätzlichen heterocyclischen Kuppler, der ausgewählt ist unter:
(i) den Indolderivaten der folgenden Formel (II) und deren Additionssalzen mit einer Säure: 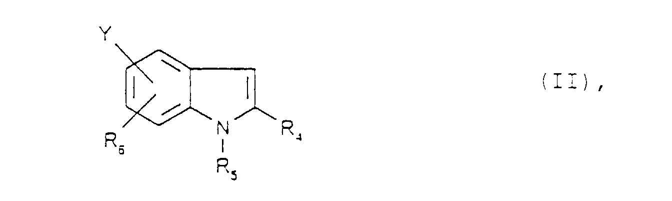 worin bedeuten:
R₄ und R₅, die identisch oder voneinander verschieden sind, Wasserstoff oder C₁₋₄-Alkyl
R₆ Wasserstoff, C₁₋₄-Alkyl oder Hydroxy, und
Y Hydroxy oder NHR₇, wobei R₇ Wasserstoff, C₁₋₄-Alkyl oder C₁₋₄-Hydroxyalkyl bedeutet, mit der Maßgabe, daß:
- wenn R₆ Hydroxy bedeutet, R₆ in 6-Stellung substituiert ist, Y Hydroxy bedeutet und in 5-Stellung substituiert ist, und R₄ und R₅ Wasserstoff bedeuten;
- wenn Y Hydroxy bedeutet, Y in 6- oder 7-Stellung substituiert ist und R₆ von Hydroxy verschieden ist,
- wenn Y Amino bedeutet, Y in 4-, 6- oder 7-Stellung substituiert ist;
(ii) den Benzimidazolderivaten der folgenden Formel (III) und deren Additionssalzen mit einer Säure: 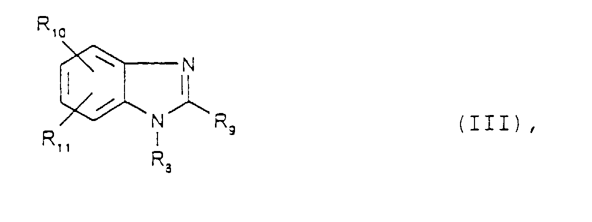 worin bedeuten:
R₈ Wasserstoff oder C₁₋₄-Alkyl,
R₉ Wasserstoff, C₁₋₄-Alkyl oder Phenyl,
R₁₀ Hydroxy, Amino oder Methoxy, und
R₁₁ Wasserstoff, Hydroxy, Methoxy oder C₁₋₄-Alkyl,
mit der Maßgabe, daß
- R₁₀ in 4-Stellung substituiert ist, wenn R₁₀ Amino bedeutet,
- R₁₁ in 7-Stellung substituiert ist, wenn R₁₀ in 4-Stellung substituiert ist, und
- R₁₁ in 6-Stellung substituiert ist, wenn R₁₀ in 5-Stellung substituiert ist;
(iii) den Benzomorpholinderivaten der folgenden Formel (IV) und deren Additionssalzen mit einer Säure: 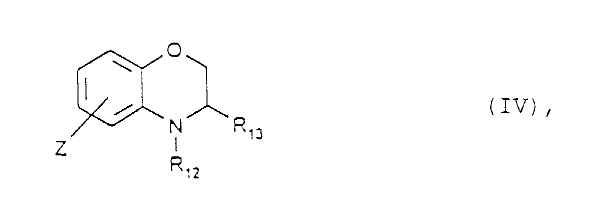 worin bedeuten:
R₁₂ und R₁₃, die identisch oder voneinander verschieden sind, Wasserstoff oder C₁₋₄-Alkyl, und
Z Hydroxy oder Amino;
(iv) den Pyridinderivaten der folgenden Formel (V) und deren Additionssalzen mit einer Säure: 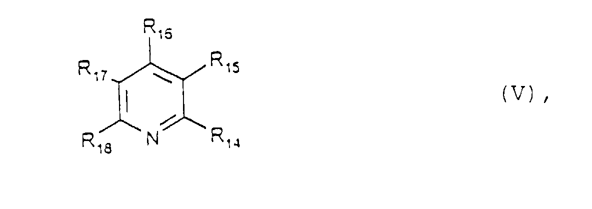 worin bedeuten:
R₁₄ Wasserstoff, Hydroxy, C₁₋₄-Alkoxy, C₁₋₄-Monohydroxyalkoxy, C₂₋₄-Polyhydroxyalkoxy, Amino oder die Gruppe -OCH₂CH₂COCH₂CH₂OH,
R₁₅ und R₁₇, die identisch oder voneinander verschieden sind, Wasserstoff, Hydroxy, Amino oder C₁₋₄-Alkyl,
R₁₆ Wasserstoff oder C₁₋₄-Alkyl, und
R₁₈ Wasserstoff, Hydroxy, C₁₋₄-Alkoxy, C₁₋₄-Monohydroxyalkoxy, C₂₋₄-Polyhydroxyalkoxy oder Amino,
mit der Maßgabe, daß
R₁₅ und R₁₇ Amino bedeuten, wenn R₁₄ eine Polyhydroxyalkoxygruppe oder die Gruppe -OCH₂CH₂COCH₂CH₂OH bedeutet,
mit der Maßgabe, daß die Verbindungen der Formel (V) nicht mehr als zwei Aminogruppen (substituiert oder nicht substituiert) oder nicht mehr als zwei Hydroxygruppen oder nicht mehr als eine Aminogruppe und eine Hydroxygruppe pro Molekül enthalten, wobei die Amino- und/oder Hydroxygruppen obligatorisch zueinander in m-Stellung substituiert sind;
(v) den Indolinderivaten, die unter 6-Hydroxyindolin, 6-Aminoindolin und 5,6-Dihydroxyindolin ausgewählt sind, und deren Additionssalze mit einer Säure,
(vi) den Chinolinderivaten der folgender Formel (VI) und deren Additionssalzen mit einer Säure: 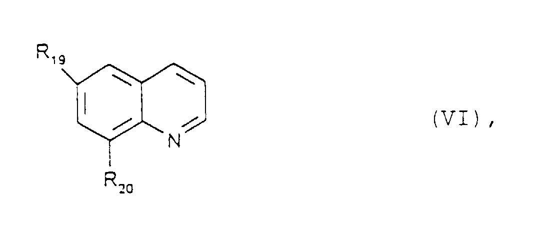 worin bedeuten:
R₁₉ Hydroxy oder C₁₋₄-Alkoxy, und
R₂₀ Wasserstoff oder Amino;
(vii) den Sesamolderivaten der folgenden Formel (VII) und deren Additionssalzen mit einer Säure: 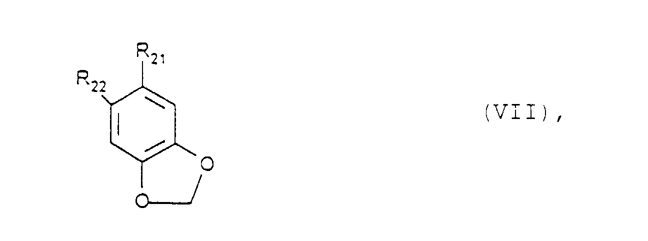 worin bedeuten:
R₂₁ Hydroxy oder Amino, und
R₂₂ Halogen oder C₁₋₄-Alkoxy.

2. Zusammensetzung nach Anspruch 1, dadurch gekennzeichnet, daß die Oxidationsbase(n) unter den p-Phenylendiaminen, Bisphenylalkylendiaminen, p-Aminophenolen, o-Aminophenolen, heterocyclischen Basen und deren Additionssalzen mit einer Säure ausgewählt sind.

3. Zusammensetzung nach Anspruch 2, dadurch gekennzeichnet, daß die p-Phenylendiamine unter den Verbindungen der folgenden Formel (VIII) und deren Additionssalzen mit einer Säure ausgewählt sind: 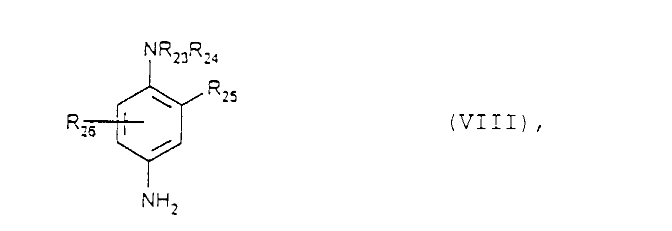 worin bedeuten:
R₂₃ Wasserstoff, C₁₋₄-Alkyl, C₁₋₄-Monohydroxyalkyl, C₂₋₄-Polyhydroxyalkyl oder C₁₋₄-Alkoxy-C₁₋₄-alkyl,
R₂₄ Wasserstoff, C₁₋₄-Alkyl, C₁₋₄-Monohydroxyalkyl oder C₂-₄-Polyhydroxyalkyl,
R₂₅ Wasserstoff, Halogen, wie Chlor, C₁₋₄-Alkyl, Sulfo, Carboxy, C₁₋₄-Monohydroxyalkyl oder C₁₋₄-Hydroxyalkoxy,
R₂₆ Wasserstoff oder C₁₋₄-Alkyl.

4. Zusammensetzung nach Anspruch 3, dadurch gekennzeichnet, daß die p-Phenylendiamine der Formel (VIII) unter p-Phenylendiamin, p-Toluylendiamin, 2-Isopropyl-p-phenylendiamin, 2-β-Hydroxyethyl-p-phenylendiamin, 2-β-Hydroxyethyloxy-p-phenylendiamin, 2,6-Dimethyl-p-phenylendiamin, 2, 6-Diethyl-p-phenylendiamin, 2,3-Dimethyl-p-phenylendiamin, N,N-Bis(β-hydroxyethyl)-p-phenylendiamin, 4-Amino-1-(β-methoxyethyl)amino-benzol, N,N'-Bis(β-hydroxyethyl)-N,N'-bis(4'-aminophenyl)-1,3-diamino-propanol, 2-Chlor-p-phenylendiamin und deren Additionssalzen mit einer Säure ausgewählt sind.

5. Zusammensetzung nach Anspruch 2, dadurch gekennzeichnet, daß die Bisphenylalkylendiamine unter den Verbindungen der folgenden Formel (IX) und deren Additionssalzen mit einer Säure ausgewählt sind: 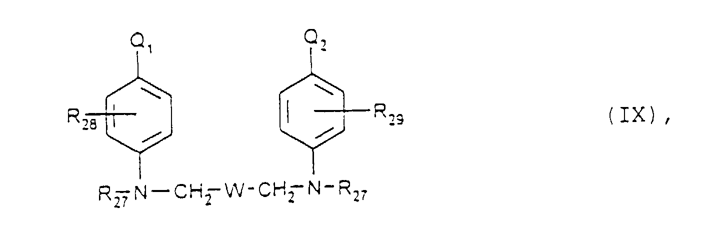 worin bedeuten:
Q₁ und Q₂, die identisch oder voneinander verschieden sind, Hydroxy oder NHR₃₀, wobei R₃₀ Wasserstoff oder C₁₋₄-Alkyl bedeutet,
R₂₇ Wasserstoff, C₁₋₄-Alkyl, C₁₋₄-Monohydroxyalkyl, C₂₋₄-Polyhydroxyalkyl oder C₁₋₄-Aminoalkyl, wobei die Aminogruppe substituiert sein kann,
R₂₈ und R₂₉, die identisch oder voneinander verschieden sind, Wasserstoff, Halogen oder C₁₋₄-Alkyl,
W eine Gruppe, die unter den folgenden Gruppen ausgewählt ist:
―(CH₂)ₙ―; ―(CH₂)ₘ―O―(CH₂)ₘ―; ―(CH₂)ₘ―CHOH―(CH₂)ₘ― und 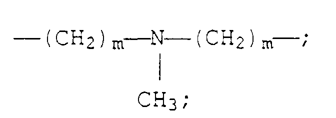 worin n Null oder eine ganze Zahl von 1 bis 8 und m Null oder eine ganze Zahl im Bereich von 1 bis 4 bedeutet.

6. Zusammensetzung nach Anspruch 5, dadurch gekennzeichnet, daß die Bisphenylalkylendiamine der Formel (IX) unter N,N'-Bis(β-hydroxyethyl)-N, N'-bis(4'-aminophenyl)-1,3-diamino-propanol, N,N'-Bis(β-hydroxyethyl)-N,N'-bis(4'-aminophenyl)-ethylendiamin, N,N'-Bis(4-aminophenyl)tetramethylendiamin, N,N'-Bis(β-hydroxyethyl)-N,N'-bis(4-aminophenyl)-tetramethylendiamin, N,N'-Bis(4-methylaminophenyl)-tetramethylendiamin, N,N'-Bis-(ethyl)-N,N'-bis(4'-amino, 3'-methylphenyl)-ethylendiamin und deren Additionssalzen mit einer Säure ausgewählt sind.

7. Zusammensetzung nach Anspruch 2, dadurch gekennzeichnet, daß die p-Aminophenole unter den Verbindungen der folgenden Formel (X) und deren Additionssalzen mit einer Säure ausgewählt sind: 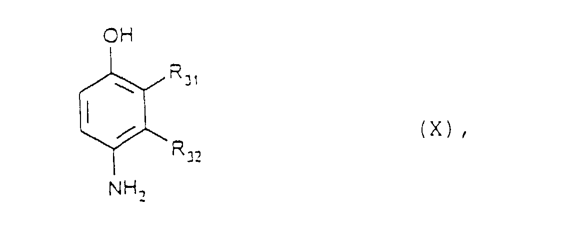 worin bedeuten:
R₃₁ Wasserstoff, C₁₋₄-Alkyl, C₁₋₄-Monohydroxyalkyl, C₁₋₄-Alkoxy-C₁₋₄-alkyl oder C₁₋₄-Aminoalkyl,
R₃₂ Wasserstoff, Fluor, C₁₋₄-Alkyl, C₁₋₄-Monohydroxyalkyl, C₂₋₄-Polyhydroxyalkyl, C₁₋₄-Aminoalkyl, C₁₋₄-Cyanoalkyl oder C₁₋₄-Alkoxy-C₁₋₄-alkyl,
mit der Maßgabe, daß mindestens eine der Gruppen R₃₁ oder R₃₂ Wasserstoff bedeutet.

8. Zusammensetzung nach Anspruch 7, dadurch gekennzeichnet, daß die p-Aminophenole der Formel (X) unter p-Aminophenol, 4-Amino-3-methyl-phenol, 4-Amino-3-fluor-phenol, 4-Amino-3-hydroxymethyl-phenol, 4-Amino-2-methyl-phenol, 4-Amino-2-hydroxymethyl-phenol, 4-Amino-2-methoxymethylphenol, 4-Amino-2-aminomethyl-phenol, 4-Amino-2-(β-hydroxyethylaminomethyl)-phenol und deren Additionssalzen mit einer Säure ausgewählt sind.

9. Zusammensetzung nach Anspruch 2, dadurch gekennzeichnet, daß die o-Aminophenole unter 2-Aminophenol, 2-Amino-1-hydroxy-5-methyl-benzol, 2-Amino-l-hydroxy-6-methylbenzol, 5-Acetamido-2-amino-phenol und deren Additionssalzen mit einer Säure ausgewählt sind.

10. Zusammensetzung nach Anspruch 2, dadurch gekennzeichnet, daß die heterocyclischen Basen unter den Pyridinderivaten, Pyrimidinderivaten, Pyrazolderivaten und deren Additionssalzen mit einer Säure ausgewählt sind.

11. Zusammensetzung nach Anspruch 10, dadurch gekennzeichnet, daß die heterocyclischen Oxidationsbasen unter 2,5-Diaminopyridin, 2,4,5,6-Tetraaminopyrimidin, 4-Hydroxy-2,5,6-triaminopyrimidin, 4,5-Diamino-1-methylpyrazol, 3,4-Diamonopyrazol und deren Additionssalzen mit einer Säure ausgewählt sind.

12. Zusammensetzung nach einem der vorhergehenden Ansprüche, dadurch gekennzeichnet, daß die Indolkuppler der Formel (I) unter 4-hydroxyindol, 4-Hydroxy-5-ethoxy-indol, 4-Hyhydroxy-5-methoxy-indol, 4-Hydroxy-1-methyl-5-ethoxyindol, 4-Hydroxy-2-ethoxycarbonyl-5-ethoxy-indol, 4-Hydroxy-2-methyl-5-ethoxy-indol, 4-Hydroxy-5-methylindol, 4-hydroxy-2-methyl-indol, 4-Hydroxy-1-methyl-indol und deren Additionssalzen mit einer Säure ausgewählt sind.

13. Zusammensetzung nach einem der vorhergehenden Ansprüche, dadurch gekennzeichnet, daß die Indolderivate der Formel (II), die als zusätzliche heterocyclische Kuppler verwendet werden, unter 6-Hydroxyindol, 7-Aminoindol, 6-Aminoindol, 7-Hydroxyindol, 7-Ethyl-6-(β-hydroxyethyl)-aminoindol, 4-Aminoindol, 6-Hydroxy-1-methyl-indol, 5,6-Dihydroxyindol und deren Additionssalzen mit einer Säure ausgewählt sind.

14. Zusammensetzung nach einem der Ansprüche 1 bis 12, dadurch gekennzeichnet, daß die Benzimidazolderivate der Formel (III), die als zusätzliche heterocyclische Kuppler verwendet werden, unter 4-Hydroxybenzimidazol, 4-Aminobenzimidazol, 4-Hydroxy-7-methyl-benzimidazol, 4-Hydroxy-2-methyl-benzimidazol, 1-Butyl-4-hydroxy-benzimidazol, 4-Amino-2-methyl-benzimidazol, 5,6-Dihydroxybenzimidazol, 5-Hydroxy-6-methoxy-benzimidazol, 4,7-Dihydroxybenzimidazol, 4,7-Dihydroxy-1-methyl-benzimidazol, 4,7-Dimethoxybenzimidazol, 5,6-Dihydroxy-1-methylbenzimidazol, 5,6-Dihydroxy-2-methyl-benzimidazol, 5,6-Dimethoxybenzimidazol und deren Additionssalzen mit einer Säure ausgewählt sind.

15. Zusammensetzung nach einem der Ansprüche 1 bis 12, dadurch gekennzeichnet, daß die Benzomorpholinderivate der Formel (IV), die als zusätzliche heterocyclische Kuppler verwendet werden, unter 6-Hydroxybenzomorpholin, N-Methyl-6-hydroxy-benzomorpholin, 6-Aminobenzomorpholin und deren Additionssalzen mit einer Säure ausgewählt sind.

16. Zusammensetzung nach einem der Ansprüche 1 bis 12, dadurch gekennzeichnet, daß die Pyridinderivate der Formel (V), die als zusätzliche heterocyclische Kuppler verwendet werden, unter 2,6-Dihydroxy-4-methyl-pyridin, 2,6-Dihydroxy-3,4-dimethyl-pyridin, 3,5-Diamino-2,6-dimethoxypyridin, 2,6-Bis(β-hydroxyethyl)oxy-3,5-diamino-pyridin, 3-Amino-2,6-dimethoxy-5-hydroxy-pyridin, 2,6-Diaminopyridin, 3-Oxo-5-(3',5'-diamino-2'-pyridyloxy)-pentanol, 3-(3',5'-Diamino-2'-pyridyloxy)-2-hydroxy-propanol und deren Additionssalzen mit einer Säure ausgewählt sind.

17. Zusammensetzung nach einem der Ansprüche 1 bis 12, dadurch gekennzeichnet, daß die Chinolinderivate der Formel (VI), die als zusätzliche heterocyclische Kuppler verwendet werden, unter 6-Hydroxychinolin, 8-Amino-6-methoxychinolin und deren Additionssalzen mit einer Säure ausgewählt sind.

18. Zusammensetzung nach einem der Ansprüche 1 bis 12, dadurch gekennzeichnet, daß die Sesamolderivate der Formel (VII), die als zusätzliche heterocyclische Kuppler verwendet werden, unter 2-Brom-4,5-methylendioxy-phenol, 1-Amino-6-methoxy-3,4-methylendioxy-benzol und deren Additionssalzen mit einer Säure ausgewählt sind.

19. Zusammensetzung nach einem der vorhergehenden Ansprüche, dadurch gekennzeichnet, daß die Additionssalze mit einer Säure unter den Hydrochloriden, Hydrobromiden, Sulfaten und Tartraten ausgewählt sind.

20. Zusammensetzung nach einem der vorhergehenden Ansprüche, dadurch gekennzeichnet, daß sie mindestens eine der folgenden ternären Kombinationen enthält:
(a): p-Phenylendiamin,
(b): 4-Hydroxyindol,
(c): 6-Hydroxyindol;
(a): p-Phenylendiamin,
(b): 4-Hydroxyindol,
(c): 4-Hydroxybenzimidazol;
(a): p-Phenylendiamin,
(b): 4-Hydroxyindol,
(c): 6-Hydroxybenzmorpholin;
(a): p-Aminophenol,
(b): 4-Hydroxyindol,
(c): 6-Hydroxyindol;
(a): 4-Amino-3-methylphenol,
(b): 4-Hydroxyindol,
(c): 6-Hydroxyindol;
(a): 2-Aminophenol,
(b): 4-Hydroxyindol,
(c): 6-Hydroxyindol;
(a): 4,5-Diamino-1-methylpyrazol,
(b): 4-Hydroxyindol,
(c): 6-Hydroxyindol;
(a): 4,5-Diamino-1-methylpyrazol,
(b): 4-Hydroxyindol,
(c): 4-Hydroxybenzimidazol;
(a): 4,5-Diamino-1-methylpyrazol,
(b): 4-Hydroxyindol,
(c): 6-Hydroxybenzomorpholin;
(a): 2,6-Dimethyl-p-phenylendiamin,
(b): 4-Hydroxyindol,
(c): 6-Hydroxyindol;
(a): 2,6-Dimethyl-p-phenylendiamin,
(b): 4-Hydroxyindol,
(c): 4-Hydroxybenzimidazol;
(a): 2,6-Dimethyl-p-phenylendiamin,
(b): 4-Hydroxyindol,
(c): 6-Hydroxybenzomorpholin;
(a): 2-β-Hydroxyethyl-p-phenylendiamin,
(b): 4-Hydroxyindol,
(c): 6-Hydroxyindol;
(a): 2-β-Hydroxyethyl-p-phenylendiamin,
(b): 4-Hydroxyindol,
(c): 4-Hydroxybenzimidazol;
(a): p-Phenylendiamin,
(b): 4-Hydroxy-1-N-methylindol,
(c): 6-Hydroxyindol;
(a): p-Phenylendiamin,
(b): 4-Hydroxy-2-methylindol,
(c): 6-Hydroxyindol;
(a): p-Aminophenol,
(b): 4-Hydroxy-1-N-methylindol,
(c): 6-Hydroxyindol;
(a): p-Aminophenol,
(b): 4-Hydroxy-2-methylindol,
(c): 6-Hydroxyindol;
(a): 4-Amino-3-methylphenol,
(b): 4-Hydroxy-1-N-methylindol,
(c): 6-Hydroxyindol;
(a) : 4-Amino-3-methylphenol,
(b): 4-Hydroxy-2-methylindol,
(c): 6-Hydroxyindol;
(a): p-Toluylendiamin,
(b): 4-Hydroxyindol,
(c): 6-Hydroxyindol;
(a): p-Toluylendiamin,
(b): 4-Hydroxyindol,
(c): 4-Hydroxybenzimidazol;
(a): p-Toluylendiamin,
(b): 4-Hydroxyindol,
(c): 6-Hydroxybenzomorpholin;
(a): 4-Amino-3-methylphenol,
(b): 4-Hydroxyindol,
(c): 6-Hydroxybenzomorpholin;
(a): 2,6-Dimethyl-p-phenylendiamin,
(b): 4-Hydroxy-1-methylindol,
(c): 1-Amino-6-methoxy-3,4-methylendioxybenzol;
(a): 3,4-Diaminopyrazol,
(b): 4-Hydroxyindol,
(c): 2, 6-Diaminopyridin.

21. Zusammensetzung nach einem der vorhergehenden Ansprüche, dadurch gekennzeichnet, daß die Oxidationsbasen insgesamt 0,0005 bis 12 Gew.-% des Gesamtgewichts der Färbemittelzusammensetzung ausmachen.

22. Zusammensetzung nach Anspruch 21, dadurch gekennzeichnet, daß die Oxidationsbasen insgesamt 0,005 bis 6 Gew.-% des Gesamtgewichts der Färbemittelzusammensetzung ausmachen.

23. Zusammensetzung nach einem der vorhergehenden Ansprüche, dadurch gekennzeichnet, daß der oder die Indolkuppler der Formel (I) 0,0001 bis 5 Gew.-%, des Gesamtgewichts der Färbemittelzusammensetzung ausmachen.

24. Zusammensetzung nach Anspruch 23, dadurch gekennzeichnet, daß der oder die Indolkuppler der Formel (I) 0,005 bis 3 Gew.-% des Gesamtgewichts der Färbemittelzusammensetzung ausmachen.

25. Zusammensetzung nach einem der vorhergehenden Ansprüche, dadurch gekennzeichnet, daß der oder die zusätzlichen heterocyclischen Kuppler 0,0001 bis 10 Gew.-% des Gesamtgewichts der Färbemittelzusammensetzung ausmachen.

26. Zusammensetzung nach Anspruch 25, dadurch gekennzeichnet, daß der oder die zusätzlichen heterocyclischen Kuppler 0,005 bis 5 Gew.-% des Gesamtgewichts der Färbemittelzusammensetzung ausmachen.

27. Zusammensetzung nach einem der vorhergehenden Ansprüche, dadurch gekennzeichnet, daß das zum Färben geeignete Medium (oder der Träger) aus Wasser oder einem Gemisch von Wasser und mindestens einem organischen Lösungsmittel besteht, das unter den niederen C₁₋₄-Alkanolen, Glycerin, Glykolen und Glykolethern, aromatischen Alkoholen, analogen Produkten und deren Gemischen ausgewählt ist.

28. Zusammensetzung nach einem der vorhergehenden Ansprüche, dadurch gekennzeichnet, daß sie einen pH-Wert im Bereich von 3 bis 12 aufweist.

29. Verfahren zum Färben von Keratinfasern und insbesondere menschlichen Keratinfasern, wie dem Haar, dadurch gekennzeichnet, daß auf die Fasern eine Färbemittelzusammensetzung nach einem der Ansprüche 1 bis 28 aufgetragen wird und die Farbe bei einem sauren, neutralen oder alkalischen pH-Wert mit einem Oxidationsmittel entwickelt wird, das bei der Anwendung zu der Färbemittelzusammensetzung gegeben wird oder das in einer oxidierenden Zusammensetzung vorliegt, die gleichzeitig oder getrennt davon anschließend aufgetragen wird.

30. Verfahren nach Anspruch 29, dadurch gekennzeichnet, daß das Oxidationsmittel unter Wasserstoffperoxid, Harnstoffperoxid, Alkalimetallbromaten und Salzen von Persäuren, wie Perboraten und Persulfaten, ausgewählt ist.

31. Vorrichtung mit mehreren Abteilungen oder "Kit" zum Färben mit mehreren Abteilungen, wobei eine erste Abteilung eine Färbemittelzusammensetzung nach einem der Ansprüche 1 bis 28 und eine zweite Abteilung eine oxidierende Zusammensetzung enthält.

## Claims

1. Composition for the oxidation dyeing of keratin fibres, and in particular human keratin fibres such as the hair, characterized in that it comprises, in a medium which is suitable for dyeing:
(a) at least one oxidation base,
(b) at least one indole coupler of following formula (I) and/or at least one of the addition salts thereof with an acid: 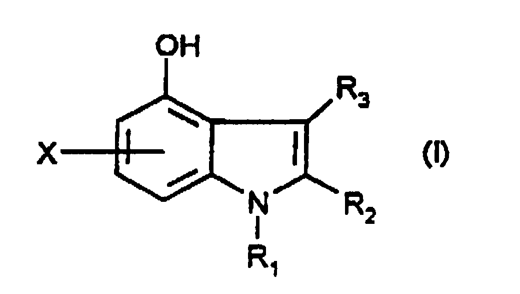 in which:
R₁ represents a hydrogen atom or a C₁-C₄ alkyl radical,
R₂ and R₃, which may be identical or different, represent a hydrogen atom or a C₁-C₄ alkyl, carboxyl or (C₁-C₄) alkoxycarbonyl radical,
X represents a hydrogen or halogen atom or a C₁-C₄ alkyl, C₁-C₁₈ alkoxy or acetylamino radical;
(c) at least one additional heterocyclic coupler chosen from:
(i) the indole derivatives of following formula (II), and the addition salts thereof with an acid: 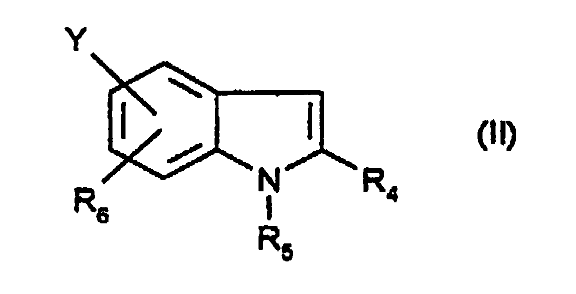 in which:
R₄ and R₅, which may be identical or different, represent a hydrogen atom or a C₁-C₄ alkyl radical; R₆ represents a hydrogen atom or a C₁-C₄ alkyl or hydroxyl radical;
Y represents a hydroxyl radical or a radical NHR₇ in which R₇ represents a hydrogen atom or a C₁-C₄ alkyl or C₁-C₄ hydroxyalkyl radical;
with the proviso that :
- when R₆ denotes hydroxyl, it then occupies the 6-position, Y denotes hydroxyl and occupies the 5-position and R₄ and R₅ represent a hydrogen atom,
- when Y denotes hydroxyl, it then occupies the 6- or 7-position, and R₆ is other than hydroxyl,
- when Y denotes amino, it then occupies the 4-, 6- or 7-position;
(ii) the benzimidazole derivatives of following formula (III), and the addition salts thereof with an acid: 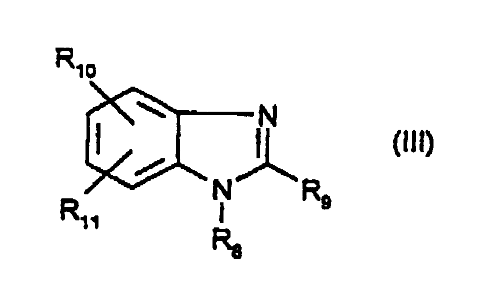 in which:
R₈ represents a hydrogen atom or a C₁-C₄ alkyl radical,
R₉ represents a hydrogen atom or a C₁-C₄ alkyl or phenyl radical,
R₁₀ represents a hydroxyl, amino or methoxy radical,
R₁₁ represents a hydrogen atom or a hydroxyl, methoxy or C₁-C₄ alkyl radical;
with the proviso that:
- when R₁₀ denotes an amino radical, it then occupies the 4-position,
- when R₁₀ occupies the 4-position, R₁₁ then occupies the 7-position,
- when R₁₀ occupies the 5-position, R₁₁ then occupies the 6-position;
(iii) the benzomorpholine derivatives of following formula (IV), and the addition salts thereof with an acid: 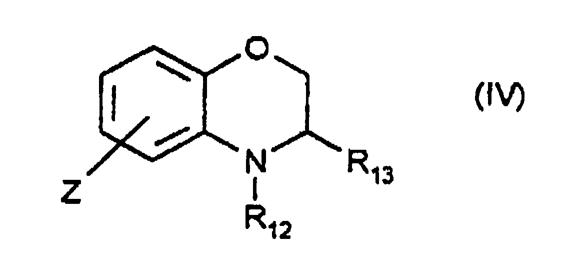 in which:
R₁₂ and R₁₃, which may be identical or different, represent a hydrogen atom or a C₁-C₄ alkyl radical,
Z represents a hydroxyl or amino radical;
(iv) the pyridine derivatives of following formula (V), and the addition salts thereof with an acid: 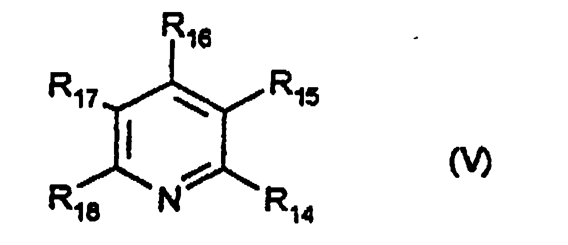 in which:
R₁₄ represents a hydrogen atom, a hydroxyl, C₁-C₄ alkoxy, C₁-C₄ monohydroxyalkoxy, C₂-C₄ polyhydroxyalkoxy or amino radical or the -OCH₂CH₂COCH₂CH₂OH group,
R₁₅ and R₁₇, which may be identical or different, represent a hydrogen atom or a hydroxyl, amino or C₁-C₄ alkyl radical,
R₁₆ represents a hydrogen atom or a C₁-C₄ alkyl radical,
R₁₈ represents a hydrogen atom or a hydroxyl, C₁-C₄ alkoxy, C₁-C₄ monohydroxyalkoxy, C₂-C₄ polyhydroxyalkoxy or amino radical,
with the proviso that when R₁₄ represents a polyhydroxyalkoxy radical or the -OCH₂CH₂COCH₂CH₂OH group, R₁₅ and R₁₇ then represent an amino radical; and it being lastly understood that these compounds of formula (V) contain not more than two amino groups (substituted or unsubstituted) or not more than two hydroxyl groups or not more than one amino group and one hydroxyl group per molecule, these amino and/or hydroxyl groups necessarily being in a meta position relative to each other;
(v) the indoline derivatives chosen from 6-hydroxyindoline, 6-aminoindoline and 5,6-dihydroxyindoline, and the addition salts thereof with an acid;
(vi) the quinoline derivatives of following formula (VI), and the addition salts thereof with an acid: 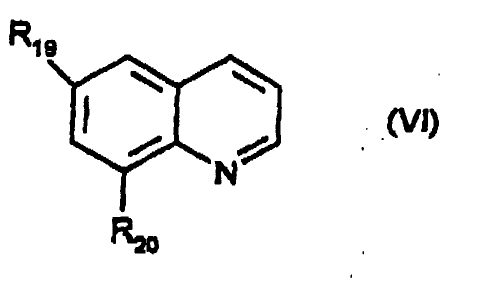 in which:
R₁₉ denotes a hydroxyl or C₁-C₄ alkoxy radical,
R₂₀ denotes a hydrogen atom or amino radical;
(vii) the sesamol derivatives of following formula (VII), and the addition salts thereof with an acid: 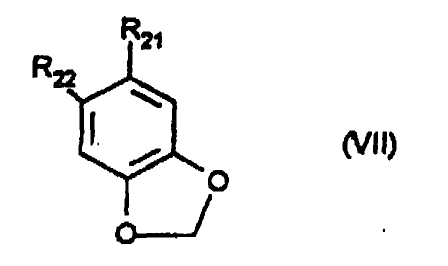 in which:
R₂₁ denotes a hydroxyl or amino radical,
R₂₂ denotes a halogen atom or a C₁-C₄ alkoxy radical.

2. Composition according to Claim 1, characterized in that the oxidation base or bases are chosen from para-phenylenediamines, bis (phenyl)alkylenediamines, para-aminophenols, ortho-aminophenols and heterocyclic bases, and the addition salts thereof with an acid.

3. Composition according to Claim 2, characterized in that the para-phenylenediamines are chosen from the compounds corresponding to the following formula (VIII), and the addition salts thereof with an acid: 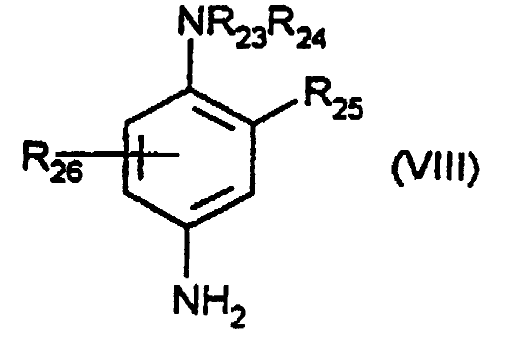 in which:
R₂₃ represents a hydrogen atom or a C₁-C₄ alkyl, C₁-C₄ monohydroxyalkyl, C₂-C₄ polyhydroxyalkyl or (C₁-C₄) alkoxy (C₁-C₄) alkyl radical,
R₂₄ represents a hydrogen atom or a C₁-C₄ alkyl, C₁-C₄ monohydroxyalkyl or C₂-C₄ polyhydroxyalkyl radical,
R₂₅ represents a hydrogen atom, a halogen atom such as a chlorine atom, or a C₁-C₄ alkyl, sulpho, carboxyl, C₁-C₄ monohydroxyalkyl or C₁-C₄ hydroxyalkoxy radical,
R₂₆ represents a hydrogen atom or a C₁-C₄ alkyl radical.

4. Composition according to Claim 3, characterized in that the para-phenylenediamines of formula (VIII) are chosen from para-phenylenediamine, para-toluylenediamine, 2-isopropyl-para-phenylenediamine, 2-β-hydroxyethyl-para-phenylenediamine, 2-β-hydroxyethyloxy-para-phenylenediamine, 2,6-dimethyl-paraphenylenediamine, 2,6-diethyl-para-phenylenediamine, 2,3-dimethyl-para-phenylenediamine, N,N-bis(β-hydroxyethyl)-para-phenylenediamine, 4-amino-1-(β-methoxyethyl)amino-benzene, N,N'-bis(β-hydroxyethyl)-N,N'-bis(4'-aminophenyl)-1,3-diamino-2-propanol, 2-chloro-para-phenylenediamine, and the addition salts thereof with an acid.

5. Composition according to Claim 2, characterized in that the bis(phenyl)alkylenediamines are chosen from the compounds corresponding to the following formula (IX), and the addition salts thereof with an acid: 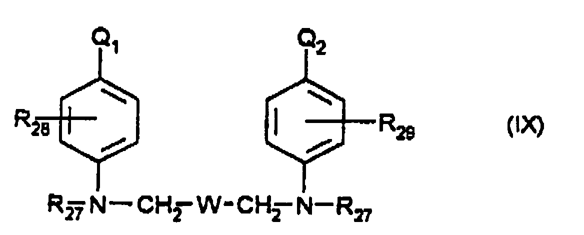 in which:
Q₁ and Q₂, which may be identical or different, represent a hydroxyl radical or a radical NHR₃₀ in which R₃₀ represents a hydrogen atom or a C₁-C₄ alkyl radical,
R₂₇ represents a hydrogen atom or a C₁-C₄ alkyl, C₁-C₄ monohydroxyalkyl or C₂-C₄ polyhydroxyalkyl radical or a C₁-C₄ aminoalkyl radical in which the amino residue may be substituted,
R₂₈ and R₂₉, which may be identical or different, represent a hydrogen or halogen atom or a C₁-C₄ alkyl radical,
W represents a radical taken from the group consisting of the following radicals:
- (CH₂)ₙ-; -(CH₂)ₘ-O-(CH₂)ₘ-; -(CH₂)ₘ-CHOH-(CH₂)ₘ- and 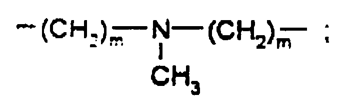 in which n is an integer between 0 and 8 inclusive and m is an integer between 0 and 4 inclusive.

6. Composition according to Claim 5, characterized in that the bis(phenyl)alkylenediamines of formula (IX) are chosen from N,N'-bis(β-hydroxyethyl)-N,N'-bis(4'-aminophenyl)-1,3-diaminopropanol, N,N'-bis(β-hydroxyethyl)-N,N'-bis(4'-aminophenyl)ethylenediamine, N,N'-bis(4-aminophenyl)tetramethylenediamine, N,N'-bis(β-hydroxyethyl)-N,N'-bis(4-aminophenyl)tetramethylenediamine, N,N'-bis(4-methylaminophenyl)tetramethylenediamine, N,N'-bis(ethyl)-N,N'-bis(4'-amino-3'-methylphenyl)ethylenediamine, and the addition salts thereof with an acid.

7. Composition according to Claim 2, characterized in that the para-aminophenols are chosen from the compounds corresponding to the following formula (X), and the addition salts thereof with an acid: 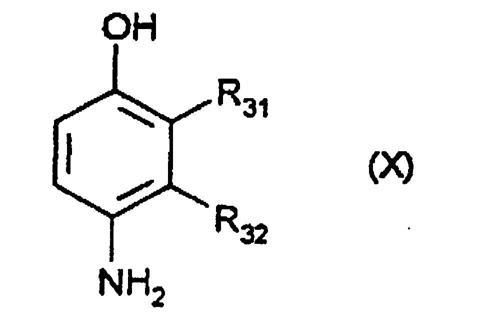 in which:
R₃₁ represents a hydrogen atom or a C₁-C₄ alkyl, C₁-C₄ monohydroxyalkyl, (C₁-C₄)alkoxy(C₁-C₄)alkyl or C₁-C₄ aminoalkyl radical,
R₃₂ represents a hydrogen or fluorine atom or a C₁-C₄ alkyl, C₁-C₄ monohydroxyalkyl, C₂-C₄ polyhydroxyalkyl, C₁-C₄ aminoalkyl, C₁-C₄ cyanoalkyl or (C₁-C₄)alkoxy(C₁-C₄) alkyl radical,
it being understood that at least one of the radicals R₃₁ or R₃₂ represents a hydrogen atom.

8. composition according to Claim 7, characterized in that the para-aminophenols of formula (X) are chosen from para-aminophenol, 4-amino-3-methylphenol, 4-amino-3-fluorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol, 4-amino-2-(β-hydroxyethyl-aminomethyl)phenol, and the addition salts thereof with an acid.

9. Composition according to Claim 2, characterized in that the ortho-aminophenols are chosen from 2-aminophenol, 2-amino-1-hydroxy-5-methylbenzene, 2-amino-1-hydroxy-6-methylbenzene, 5-acetamido-2-aminophenol, and the addition salts thereof with an acid.

10. Composition according to Claim 2, characterized in that the heterocyclic bases are chosen from pyridine derivatives, pyrimidine derivatives and pyrazole derivatives, and the addition salts thereof with an acid.

11. Composition according to Claim 10, characterized in that the heterocyclic oxidation bases are chosen from 2,5-diaminopyridine, 2,4,5,6-tetraaminopyrimidine, 4-hydroxy-2,5,6-triamino-pyrimidine, 4,5-diamino-1-methylpyrazole and 3,4-diaminopyrazole, and the addition salts thereof with an acid.

12. Composition according to any one of the preceding claims, characterized in that the indole couplers of formula (I) are chosen from 4-hydroxyindole, 4-hydroxy-5-ethoxyindole, 4-hydroxy-5-methoxyindole, 4-hydroxy-1-methyl-5-ethoxyindole, 4-hydroxy-2-ethoxycarbonyl-5-ethoxyindole, 4-hydroxy-2-methyl-5-ethoxyindole, 4-hydroxy-5-methylindole, 4-hydroxy-2-methylindole, 4-hydroxy-1-methylindole, and the addition salts thereof with an acid.

13. Composition according to any one of the preceding claims, characterized in that the indole derivatives of formula (II), used as additional heterocyclic couplers, are chosen from 6-hydroxyindole, 7-aminoindole, 6-aminoindole, 7-hydroxyindole, 7-ethyl-6-(β-hydroxyethyl)aminoindole, 4-aminoindole, 6-hydroxy-1-methylindole and 5,6-dihydroxyindole, and the addition salts thereof with an acid.

14. Composition according to any one of Claims 1 to 12, characterized in that the benzimidazole derivatives of formula (III), used as additional heterocyclic couplers, are chosen from 4-hydroxy-benzimidazole, 4-aminobenzimidazole, 4-hydroxy-7-methylbenzimidazole, 4-hydroxy-2-methylbenzimidazole, 1-butyl-4-hydroxybenzimidazole, 4-amino-2-methylbenzimidazole, 5,6-dihydroxybenzimidazole, 5-hydroxy-6-methoxybenzimidazole, 4,7-dihydroxy-benzimidazole, 4,7-dihydroxy-1-methylbenzimidazole, 4,7-dimethoxybenzimidazole, 5,6-dihydroxy-1-methyl-benzimidazole, 5,6-dihydroxy-2-methylbenzimidazole and 5,6-dimethoxybenzimidazole, and the addition salts thereof with an acid.

15. Composition according to any one of Claims 1 to 12, characterized in that the benzomorpholine derivatives of formula (IV), used as additional heterocyclic couplers, are chosen from 6-hydroxybenzomorpholine, N-methyl-6-hydroxybenzomorpholine and 6-aminobenzomorpholine, and the addition salts thereof with an acid.

16. Composition according to any one of Claims 1 to 12, characterized in that the pyridine derivatives of formula (V), used as additional heterocyclic couplers, are chosen from 2,6-dihydroxy-4-methylpyridine, 2,6-dihydroxy-3,4-dimethylpyridine, 3,5-diamino-2,6-dimethoxypyridine, 2,6-bis(β-hydroxyethyl)oxy-3,5-diaminopyridine, 3-amino-2,6-dimethoxy-5-hydroxypyridine, 2,6-diaminopyridine, 3-oxo-5-(3',5'-diamino-2'-pyridyloxy)pentanol and 3-(3',5'-diamino-2'-pyridyloxy)-2-hydroxypropanol, and the addition salts thereof with an acid.

17. Composition according to any one of Claims 1 to 12, characterized in that the quinoline derivatives of formula (VI), used as additional heterocyclic couplers, are chosen from 6-hydroxy-quinoline and 8-amino-6-methoxyquinoline, and the addition salts thereof with an acid.

18. Composition according to any one of Claims 1 to 12, characterized in that the sesamol derivatives of formula (VII), used as additional heterocyclic couplers, are chosen from 2-bromo-4,5-methylenedioxyphenol and 1-amino-6-methoxy-3,4-methylenedioxybenzene, and the addition salts thereof with an acid.

19. Composition according to any one of the preceding claims, characterized in that the addition salts with an acid are chosen from the hydrochlorides, hydrobromides, sulphates and tartrates.

20. Composition according to one of the preceding claims, characterized in that it includes at least one of the following ternary combinations:
(a): para-phenylenediamine,
(b): 4-hydroxyindole,
(c): 6-hydroxyindole;
(a): para-phenylenediamine,
(b): 4-hydroxyindole,
(c): 4-hydroxybenzimidazole;
(a): para-phenylenediamine,
(b): 4-hydroxyindole,
(c): 6-hydroxybenzomorpholine;
(a): para-aminophenol,
(b): 4-hydroxyindole,
(c): 6-hydroxyindole;
(a): 4-amino-3-methylphenol,
(b): 4-hydroxyindole,
(c): 6-hydroxyindole;
(a): 2-aminophenol,
(b): 4-hydroxyindole,
(c): 6-hydroxyindole;
(a): 4,5-diamino-1-methylpyrazole,
(b): 4-hydroxyindole,
(c): 6-hydroxyindole;
(a): 4,5-diamino-1-methylpyrazole,
(b): 4-hydroxyindole,
(c): 4-hydroxybenzimidazole;
(a): 4,5-diamino-1-methylpyrazole,
(b): 4-hydroxyindole,
(c): 6-hydroxybenzomorpholine;
(a): 2,6-dimethyl-para-phenylenediamine,
(b): 4-hydroxyindole,
(c): 6-hydroxyindole;
(a): 2,6-dimethyl-para-phenylenediamine,
(b): 4-hydroxyindole,
(c): 4-hydroxybenzimidazole;
(a): 2, 6-dimethyl-para-phenylenediamine,
(b): 4-hydroxyindole,
(c): 6-hydroxybenzomorpholine,
(a): 2-p-hydroxyethyl-para-phenylenediamine,
(b): 4-hydroxyindole,
(c): 6-hydroxyindole;
(a): 2-β-hydroxyethyl-para-phenylenediamine,
(b): 4-hydroxyindole,
(c): 4-hydroxybenzimidazole;
(a): para-phenylenediamine,
(b): 4-hydroxy-1-N-methylindole,
(c): 6-hydroxyindole;
(a): para-phenylenediamine,
(b): 4-hydroxy-2-methylindole,
(c): 6-hydroxyindole;
(a): para-aminophenol,
(b): 4-hydroxy-1-N-methylindole,
(c): 6-hydroxyindole;
(a): para-aminophenol,
(b): 4-hydroxy-2-methylindole,
(c) : 6-hydroxyindole;
(a): 4-amino-3-methylphenol,
(b): 4-hydroxy-1-N-methylindole,
(c): 6-hydroxyindole;
(a): 4-amino-3-methylphenol,
(b): 4-hydroxy-2-methylindole,
(c): 6-hydroxyindole;
(a): para-toluylenediamine,
(b): 4-hydroxyindole,
(c): 6-hydroxyindole;
(a): para-toluylenediamine,
(b): 4-hydroxyindole,
(c): 4-hydroxybenzimidazole;
(a): para-toluylenediamine,
(b): 4-hydroxyindole,
(c): 6-hydroxybenzomorpholine;
(a): 4-amino-3-methylphenol,
(b): 4-hydroxyindole,
(c): 6-hydroxybenzomorpholine;
(a): 2,6-dimethyl-para-phenylenediamine,
(b): 4-hydroxy-1-methylindole,
(c): 1-amino-6-methoxy-3,4-methylenedioxybenzene;
(a): 3,4-diaminopyrazole,
(b): 4-hydroxyindole,
(c): 2,6-diaminopyridine.

21. Composition according to any one of the preceding claims, characterized in that the oxidation bases together represent from 0.0005 to 12 % by weight relative to the total weight of the dye composition.

22. Composition according to Claim 21, characterized in that the oxidation bases together represent from 0.005 to 6 % by weight relative to the total weight of the dye composition.

23. Composition according to any one of the preceding claims, characterized in that the indole coupler or couplers of formula (I) represent from 0.0001 to 5 % by weight relative to the total weight of the dye composition.

24. Composition according to Claim 23, characterized in that the indole coupler or couplers of formula (I) represent from 0.005 to 3 % by weight relative to the total weight of the dye composition.

25. Composition according to any one of the preceding claims, characterized in that the additional heterocyclic coupler or couplers represent from 0.0001 to 10 % by weight relative to the total weight of the dye composition.

26. Composition according to Claim 25, characterized in that the additional heterocyclic coupler or couplers represent from 0.005 to 5 % by weight relative to the total weight of the dye composition.

27. Composition according to any one of the preceding claims, characterized in that the medium which is suitable for dyeing (or the support) consists of water or of a mixture of water and at least one organic solvent chosen from C₁-C₄ lower alkanols, glycerol, glycols and glycol ethers, aromatic alcohols, similar products and mixtures thereof.

28. Composition according to any one of the preceding claims, characterized in that it has a pH of between 3 and 12.

29. Process for dyeing keratin fibres, and in particular human keratin fibres such as the hair, characterized in that a dye composition as defined in any one of Claims 1 to 28 is applied to these fibres and in that the colour is developed at acidic, neutral or alkaline pH using an oxidizing agent which is added only at the time of use to the dye composition or which is present in an oxidizing composition that is applied simultaneously or sequentially in a separate manner.

30. Process according to Claim 29, characterized in that the oxidizing agent is chosen from hydrogen peroxide, urea peroxide, alkali metal bromates and persalts such as perborates and persulphates.

31. Multi-compartment device, or multi-compartment dyeing "kit", a first compartment of which contains a dye composition as defined in any one of Claims 1 to 28 and a second compartment of which contains an oxidizing composition.
